# EUROPEAN PATENT SPECIFICATION

(11) **EP 1 933 883 B1**
(45) Date of publication and mention of the grant of the patent: **07.10.2015**
(21) Application number: 06800819.2
(22) Date of filing: 03.08.2006
(51) Int. Cl.: A61K 49/00, G01N 33/534, G01N 33/58, G01N 33/68

(54) **METHODS AND COMPOSITIONS FOR DIAGNOSIS OF IGA-AND IGM-MEDIATED KIDNEY DISEASES**
VERFAHREN UND ZUSAMMENSETZUNGEN ZUR DIAGNOSE VON IGA- UND IGM-VERMITTELTEN NIERENERKRANKUNGEN
METHODES ET COMPOSITIONS POUR LE DIAGNOSTIC DE MALADIES RENALES MEDIEES PAR L'IGA ET L'IGM

(30) Priority: 03.08.2005 US 705282 P
(43) Date of publication of application: 25.06.2008
(73) Proprietor: Rq Bioscience, Inc., Westborough, MA 01581 (US); NEW ENGLAND MEDICAL CENTER HOSPITALS, INC., Boston, MA 02111 (US)
(72) Inventor: QIU, Robert, Sidong, Westborough, MA 01581 (US); QIU, Jiazhou, Westborough, MA 01581 (US); PLAUT, Andrew, G., Lexington, MA 02421-6340 (US)
(74) Representative: Strehl Schübel-Hopf & Partner
(86) International application number: PCT/US2006/030583
(87) International publication number: WO 2007/019376

(56) References cited:
- WO-A1-88/08983
- US-A- 4 454 106
- US-A- 5 286 850
- US-A- 5 891 628
- "MYOCARDIAL INFARCT IMAGING OF ANTIBODIES TO CANINE CARDIAC MYOSIN WITH INDIUM-111-DIETHYLENETRIAMINE PENTAACETIC ACID", SCIENCE, AMERICAN ASSOCIATION FOR THE ADVANCEMENT OF SCIENCE, WASHINGTON, DC; US, vol. 209, 11 July 1980 (1980-07-11), pages 295-297, XP009011951, ISSN: 0036-8075, DOI: DOI:10.1126/SCIENCE.7384803
- FLOEGE J ET AL: "[IgA nephropathy: frequent, but rarely diagnosed].", DER INTERNIST SEP 2003 LNKD- PUBMED:14566466, vol. 44, no. 9, September 2003 (2003-09), pages 1131-1139, XP002628952, ISSN: 0020-9554

## Description

### Background of the Invention

The invention relates to the field of compositions for use in diagnostic methods for kidney diseases and kits useful in the diagnosis of such diseases.

IgA nephropathy (IgAN, Berger's disease) is characterized by the deposition of IgA1 in the mesangium of the renal glomerulus and is the most common glomerulonephritis worldwide. The IgA deposits arise spontaneously, usually in the second or third decade of life and are thought to be immune complexes. The antigen(s) are unknown; IgA itself may be the antigen. The prevalence of the disease is high in the U.S. and Europe, but highest in Asia. The incidence in Japan may be 40-50% of all renal biopsies. Persistent or intermittently detected microscopic hematuria and proteinuria for many years is the clinical feature of this disease, and more than 50% of patients also develop hypertension. It is not a benign illness as once believed, with about 15-40% of patients eventually developing renal failure. Indeed, IgA nephropathy is the main cause of end-stage renal disease in patients with primary glomerular disease that eventually come to renal transplantation.

Henoch-Schönlein purpura (HSP) is another IgA1-mediated post-infectious vasculitis, most often in children ages 2-11 yrs, with kidney damage very similar to IgAN. Prevalance is 22/100,000 under age 14, and 70/100,000 in the group age 4 to 6 years old. HSP is so similar to IgAN that the notion that IgAN is a renal-limited form of HSP has recently gained acceptance (Smith and Feehally, Springer Semin. Immunopathol. 24:477-493,2003).

IgM nephropathy (IgMN) causes nephrotic syndrome and is characterized by IgM mesangial deposits. It is speculated that these deposits are derived from circulating IgM aggregates or immune complexes, similar to IgAN. IgMN is often seen in children, and the clinical feature of IgMN is very similar to minimal change disease (MCD), in which nephrotic syndrome is the main clinical manifestation, but IgMN results in a significantly higher incidence of hypertension then MCD.

Prior to the present invention, diagnosis of these diseases were by renal biopsy, which removes a sample of the kidney tissue for pathological examination. A renal pathologist does immunofluorescent staining of kidney tissues. This requires sequential application of anti-human IgA antibody, followed by development with a fluorochrome-conjugated second antibody to localize the IgA-based antibody-antigen complexes for the diagnosis of IgAN and HSP. In the diagnosis of IgMN, the same procedures can be applied, but the antibody used specifically binds human IgM. If the test detects high levels of glomerular IgA, more so than co-existing IgG, and with complement components variably present, the diagnosis of IgA nephropathy is made. This is the same for IgMN, in which the deposition of IgM in the glomerulus defines the illness.

Biopsy is usually done with the patient lying in the prone position, the kidney having been localized using ultrasound or CAT scan. Under local anesthesia, a small incision is made in the skin. Using an appropriate breath-holding protocol, a biopsy needle is used to take a sample the size of 1-1.5cm x 2mm, and the needle is removed. The patient remains in the hospital, lying supine for 12 to 24 hours, with monitoring to detect complications which may include bleeding, pain, arteriovenous fistula, urinary tract infection, and in rare cases, death.

Bleeding is the most common complication of renal biopsy. Although prior to biopsy patients are tested for the ability to coagulate, most patients experience microscopic hematuria for a short time. Rarely, bleeding is severe enough to require a blood transfusion or surgery. It has been estimated that surgery is required to control the bleeding in 0.1 to 0.4 percent of percutaneous renal biopsies, and removal of the kidney to control hemorrhage is required in approximately 0.06 percent (6 per 10,000) of completed needle biopsies.

Pain is a common problem and is transient. Pain lasting more than 12 hours occurs in approximately 4 percent of biopsies. Severe or prolonged pain can occur if a blood clot obstructs one of the ureters or in the event of a large subcapsular hematoma.

Arteriovenous fistula between two blood vessels can result from damage to the walls of an adjacent artery and vein caused by the biopsy needle. Such fistulas are rare and usually close spontaneously in one to two years.

Death occurs in approximately 0.1% of renal biopsy cases.

Renal biopsy is not appropriate for all patients. Contraindications include an uncorrectable bleeding condition, small kidneys, severe hypertension that cannot be medically controlled, multiple bilateral renal cysts or a renal tumor, hydronephrosis (a condition in which the flow of urine is obstructed leading to kidney damage), active infection of the tissues in or surrounding the kidney, inability of the patient to cooperate, and a solitary native kidney. Alternatives to percutaneous biopsy are the open surgery biopsy and transjugular renal biopsy

Thus there is a need for safer, more reliable, and less invasive methods for diagnosing IgA nephropathy, HSP, and IgM nephropathy.

WO 88/08983 A1 discloses a method for diagnosing IgA nephropathy wherein a substrate capable of binding fibronectin or IgA is contacted with a sample and the presence of a complex fibronectin-IgA bound to said substrate is detected.

### Summary of the Invention

The present invention features a compound for use in an in vivo method for diagnosing an IgA or IgM kidney disease in a mammal (e.g., a human) which includes administering (e.g., intravenously) to the mammal a compound which specifically binds IgA or IgM and detecting the compound in the kidney of the mammal, where an increase in the amount of the compound in the kidney of the mammal relative to the amount in the kidney of a mammal without the kidney disease is diagnostic of the IgA or IgM kidney disease in the mammal.

The present invention provides the following.
[1] A compound which specifically binds IgA or IgM for use in *in vivo* diagnosing an IgA or IgM kidney disease in a mammal, preferably a human, the compound being linked to a first member of a binding pair and to galactose, wherein the compound is detected in the kidney of said mammal by a radiolabelled substance linked to a second member of the binding pair and to galactose, wherein detection occurs following clearance of unbound compound from circulation; and an increase in the amount of said compound in the kidney of said mammal relative to the amount in the kidney of a mammal without said kidney disease is diagnostic of said IgA or IgM kidney disease in said mammal.
[2] The compound for use in the diagnosis method according to [1] above, wherein said compound comprises a peptide.
[3] The compound for use in the diagnosis method according to [2] above, wherein said peptide is selected from the group consisting of human FcαR1 (SEQ ID NO:2), human Fcα/µ receptor (SEQ ID NO:6), polymeric Ig receptor (SEQ ID NO:7), Sir22 (SEQ ID NO:3), streptococcal IgA-binding peptide (Sap; SEQ ID NO:4), a modified Z-domain protein (SEQ ID NO: 12), and YDWIPSSAW (SEQ ID NO:9), or an IgA- or IgM-binding fragment thereof.
[4] The compound for use in the diagnosis method according to [2] or [3] above, wherein said peptide comprises a modification selected from the group consisting of an N-terminal cap, a C-termina 1 cap, a D-amino acid, an amino acid surrogate, a peptidomimetic sequence, and a spacer.
[5] The compound for use in the diagnosis method according to [1] above, wherein said compound comprises an antibody, or an IgA- or IgM-binding fragment thereof, said antibody being preferably anti-human IgA, or an IgA-binding fragment thereof, or anti-human IgM, or an IgM-binding fragment thereof.
[6] The compound for use in the diagnosis method according to any one of [1] to [5] above, wherein said radiolabel on the second member of the binding pair is selected from the group consisting of ^{99m}Tc, ¹¹¹In, ⁶⁶Ga, ⁶⁷Ga, ⁶⁸Ga, ⁸⁶Y, ⁹⁰Y, ²⁰¹TI, ⁵⁵Co, ⁶⁰Cu, ⁶¹Cu, ⁶²Cu, ⁶⁴Cu, ⁶⁷Cu, ⁸²Rb, ^{185/187}Re, and ^{186/188}Re.
[7] The compound of any one of [1] to [6] above, wherein said detection is carried out by an imaging technique, preferably one of SPECT, PET, planar scan, and MRI.
[8] The compound for use in the diagnosis method according to any one of [1] to [7] above, wherein the first member of the binding pair is selected from either streptavidin or biotin.
[9] The compound for use in the diagnosis method according to any one of [1] to [8] above, wherein said administration further comprises administration of galactose-Ficoll.
[10] The compound for use in the diagnosis method according to any one of [1] to [9] above, wherein said second member of the binding pair is linked to both a galactose, and a radioactively labeled compound, said radioactively labelled compound being preferably labeled with an isotope selected from the group consisting of ^{99m}Tc, ¹¹¹In, ⁶⁶Ga, ⁶⁷Ga, ⁶⁸Ga, ⁸⁶Y, ⁹⁰Y, ²⁰¹TI, ⁵⁵Co, ⁶⁰Cu, ⁶¹Cu, ⁶²Cu, ⁶⁴Cu, ⁶⁷Cu, ⁸²Rb, ^{185/187}Re, and ^{186/188}Re.
[11] The compound for use in the diagnosis method according to [10] above, wherein said radiolabeled compound is human serum albumin.
[12] The compound for use in the diagnosis method according to any one of [1] to [11] above, wherein said second member of a binding pair is selected from either biotin or streptavidin such that it is not the same as the first member of the binding pair.
[13] A kit comprising:
   (a) an IgA- or IgM-binding compound wherein the compound is linked to a first member of a binding pair and to galactose;
   (b) a radiolabelled substance linked to a second member of the binding pair and to galactose; and
   (c) instructions for use for detecting an IgA or IgM kidney disease in a mammal.
[14] The kit of [13] above, wherein said second member of the binding pair comprises a radioactively labeled peptide, and a galactose, said radiolabel being preferably selected from the group consisting of ^{99m}Tc, ¹¹¹In, ⁶⁶Ga, ⁶⁷Ga, ⁶⁸Ga, ⁸⁶Y, ⁹⁰Y, ²⁰¹TI, ⁵⁵Co, ⁶⁰Cu, ⁶¹Cu, ⁶²Cu, ⁶⁴Cu, ⁶⁷Cu, ⁸²Rb, ^{185/187}Re, and ^{186/188}Re.
[15] The kit of [13] or [14] above, wherein said first member of the binding pair is streptavidin, and said second member of the binding pair is biotin.
[16] The kit of [13] or [14] above, wherein said radioactively labeled peptide is human serum albumin.
[17] The kit of any one of [13] to [16] above, additionally comprising galactose-Ficoll.

The compound may include a peptide (e.g., human FcaR1 (SEQ LID NO:2), human Fcα/µ receptor (SEQ ID NO:6), polymeric Ig receptor (SEQ ID NO:7), Sir22 (SEQ ID NO:3), streptococcal IgA-binding peptide (Sap; SEQ ID NO:4), a modified Z-domain protein (SEQ ID NO:12), and YDWIPSSAW (SEQ ID NO:9), or an IgA- or IgM-binding fragment of these peptides). The peptide may include a modification of an N-terminal cap, a C-terminal cap, a D-amino acid, an amino acid surrogate, a peptidomimetic sequence, or a spacer. The compound may include an antibody or an IgA- or IgM-binding fragment of an antibody (e.g., anti-human IgA or anti-human IgM). The compound may also include a qdot. The compound may be linked to a a radioactive label (e.g., ^{99m}Tc, ¹¹¹In, ⁶⁶Ga, ⁶⁷Ga, ⁶⁸Ga, ⁸⁶Y, ⁹⁰Y, ²⁰¹Tl, ⁵⁵Co, ⁶⁰Cu, ⁶¹Cu, ⁶²Cu, ⁶⁴Cu, ⁶⁷Cu, ⁸²Rb, ^{185/187}Re, and ^{186/188}Re). The linkage may be by a bifunctional chelating agent, which may include N₃S, N₂S₂, PnAO, HYNIC, [M(CO)₃(H₂O)₃]⁺, PADA, DTPA, DOTA, histidine, a tripeptide (e.g., Lys-Gly-Cys, Cys-Gly-Cys, and Gly-Gly-Cys), and a tetrapeptide (e.g., Gly-Ala-Gly-Gly or Cys-Gly-Cys-Gly). The compound may be linked to a paramagnetic substance (e.g., gadolinium). The detection may be carried out by an imaging technique (e.g., SPECT, PET, planar scan and MRI).

The compound includes a galactose and a first member of a binding pair (e.g., streptavidin). The administration of the compound may further include administration of galactose-Ficoll. The detection may then be performed by administering to the mammal a radioactively labeled (e.g., ^{99m}Tc, ¹¹¹In, ⁶⁶Ga, ⁶⁷Ga, ⁶⁸Ga, ⁸⁶Y, ⁹⁰Y, ²⁰¹Tl, ⁵⁵Co, ⁶⁰Cu, ⁶¹Cu, ⁶²Cu, ⁶⁴Cu, ⁶⁷Cu, ⁸²Rb, ^{185/187}Re, or ^{186/188}Re labeled) compound (e.g., human serum albumin) conjugated to a second member of a binding pair (e.g., biotin) and a galactose followed by detecting the radioactively labeled compound in the kidney of the mammal.

By "IgA or IgM kidney disease" is meant a disorder of the kidney mediated by IgA or IgM deposition in the kidney. These disorders include, as examples, IgA nephropathy, Henoch-Schönlein purpura, and IgM nephropathy.

By "specifically binds" is meant a compound which recognizes and binds, a compound, for example, IgA or IgM, but which does not substantially recognize and bind other molecules in a sample, for example, a biological sample, which naturally includes that compound. In one example, an antibody that specifically binds to IgA1 (SEQ ID NO:1) recognizes a 13 amino acid region present in IgA1 and absent in IgA2. Desirably, the compound binds the compound of interest, for example, IgA, at least 5-fold, 10-fold, 25-fold, 50-fold, 100-fold, or 1000-fold more strongly than it binds other components of the sample.

By "N-terminal cap" is meant any chemical modification to the amino-terminal end of a peptide or protein. In the present invention, the addition of an N-terminal cap to a peptide or protein is intended to decrease the rate of in vivo degradation of the peptide or protein as compared to the uncapped protein. Examples of N-terminal caps include acetylation and peptide cyclization.

By "C-terminal cap" is meant any chemical modification to the carboxy-terminal end of a peptide or protein. In the present invention, the addition of a C-terminal cap to a peptide or protein is intended to decrease the rate of in vivo degradation of the peptide or protein as compared to the uncapped protein. Examples of C-terminal caps include amidating or reducing the C-terminus, and peptide cyclization.

By "peptidomimetic" is meant a molecule that mimics characteristics of peptides, including the ability to recognize biomolecules. In the present invention, peptidomimetics are inserted into peptides or proteins to prevent in vivo degradation by endopeptidases and exopeptidases.

By "spacer" is meant a small molecule that is inserted in place of an amino acid in a peptide sequence either internally or at either the N- or C-termini. An examples of a spacer includes aminohexanoic acid. Like peptidomimetics, spacers are used in the present invention to prevent peptide degradation.

By "amino acid surrogate" is meant any chemical compound that can be placed into a peptide or protein in place of an amino acid. Examples of amino acid surrogates include spacers, peptidomimetics, imino acids, and amino acids with methylated amide and/or methylated side chain nitrogens.

By "fragment" is meant a chain of at least 4, 5, 6, 8, 10, 15, 20, or 25 amino acids or nucleotides which comprises any portion of a larger peptide or polynucleotide.

By "peptide" is meant any chain of amino acids, or analogs thereof, regardless of length or post-translational modification (for example, glycosylation or phosphorylation).

By "qdot" is meant a fluorescent semiconductor nanocrystal. Example of materials from which qdots are made include CdS, CdSe, CdTe, CdHgTe/ZnS, InP, InAs, and PbSe.

Other features and advantages of the invention will be apparent from the following Detailed Description, the drawings, and the claims.

### Brief Description of the Drawings

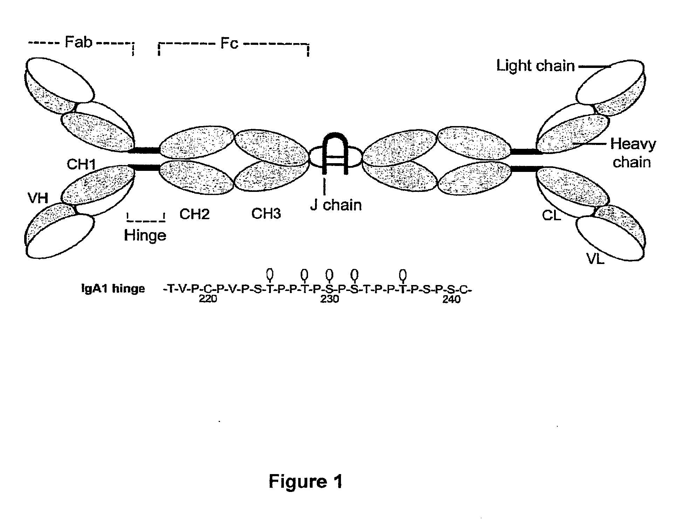

**Figure 1** is a diagram of dimeric IgA1 (SEQ ID NO:1), its hinge region (SEQ ID NO:14), and the O-glycan sites (Thr225, Thr228, Ser230, Ser232, Thr236).

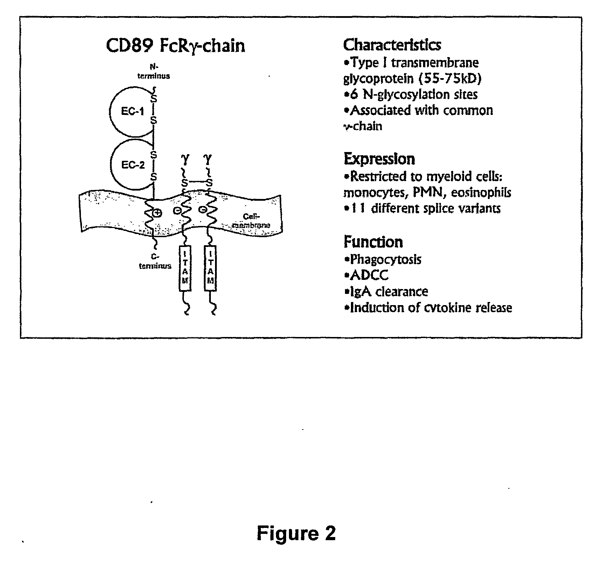

**Figure 2** is a diagram of the structure and characteristics of the Fc-receptor for IgA (FcαR1/CD89; SEQ ID NO:2). The extracellular domains 1 and 3 (EC-1 and EC-2) as well as the associated pair of γ-chains with their signaling (ITAM) motifs are depicted. Furthermore the characteristics of CD89, its cellular distribution, and its known functions are summarized. (From: Westerhuis, *Pathogenetic Aspects of IgA*-*Nephropathy* 2001 PrintPartners Ipskamp, Ensehede)

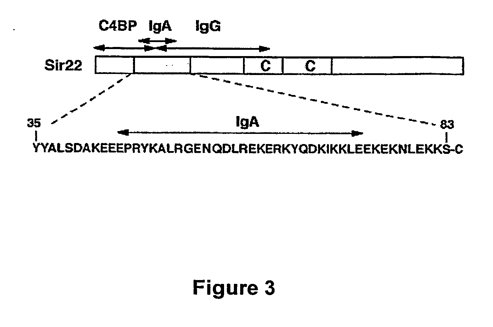

**Figure 3** is a schematic representation of the streptococcal Sir22 (M22; SEQ ID NO:3) protein and sequence of the Sap peptide (SEQ ID NO:4) derived from this protein. The Sap peptide includes the 29-residue IgA-binding region (horizontal arrows labeled "IgA") and the 10 residues on either side of this region. In addition, Sap includes a C-terminal cysteine residue not present in Sir22 that promotes its dimerization. The regions in Sir22 known to include binding sites for C4BP (the human complement regulator), IgA, and IgG are indicated. The C4BP binding region is a hypervariable domain. (The horizontal arrows under designated "C4BP" and "IgG" indicate corresponding regions for binding C4BP and IgG. There are some sequence overlaps for each of these three binding regions). The position of the conserved C repeat region is also indicated. The C-terminal end of Sir22 is covalently bound to peptidoglycan in the bacterial cell wall.

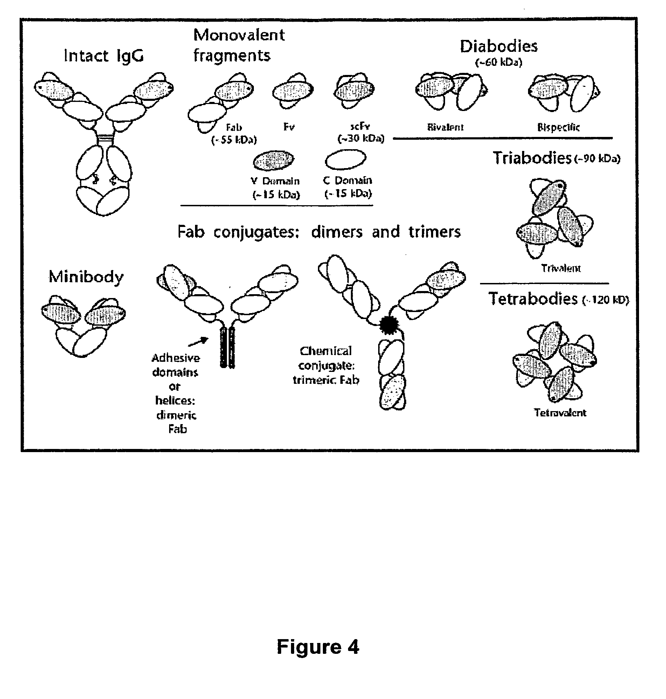

**Figure 4** is a schematic representation of an intact Ig together with Fab and Fv fragments and single V (colored ovals; dots represent antigen-binding sites) and C domains (uncolored). Engineered recombinant antibodies are shown as scFv monomers, dimers (diabodies), trimers (triabodies), and tetramers (tetrabodies), with linkers represented by a black line. Minibodies are shown as two scFv modules joined by two C domains. Also shown are Fab dimers (conjugates by adhesive polypeptide or protein domains) and Fab trimers (chemically conjugated). Colors denote different specificities for the bispecific scFv dimers (diabodies) and Fab dimers and trimers.

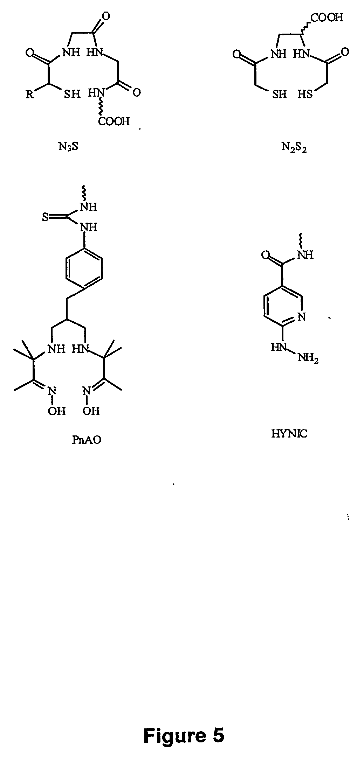

**Figure 5** is a diagram of structures of bifunctional chelating agents (BFCAs) for ^{99m}Tc. Triamidethiols (N₃S), diamidedithiols (N₂S₂), propyleneamineoxime (PnAO), and hydrazinonicotinic acid (HYNIC) are shown.

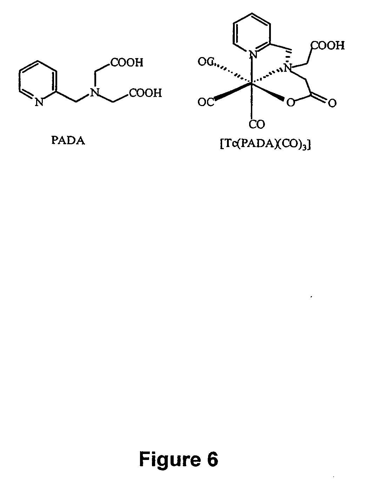

**Figure 6** is a diagram of structures of picolylamine-N,N-diacetic acid (PADA) and its complex with Tc. PADA and its complex after reaction with the aquaion [Tc(CO)₃(H₂O)₃]⁺ are shown.

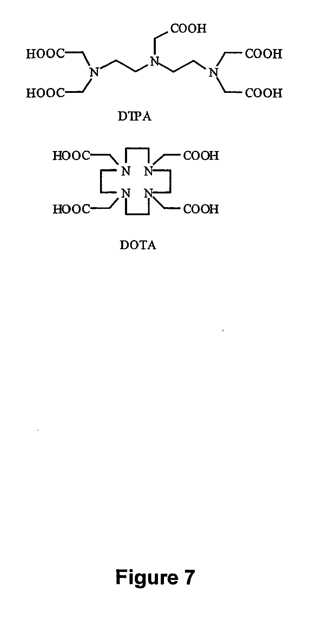

**Figure 7** is a diagram of structures of BFCAs for ¹¹¹In. Diethyl-enetriaminepentaacidic acid (DTPA) and tetraazacyclo-dodecanetetraacidic acid (DOTA) are shown.

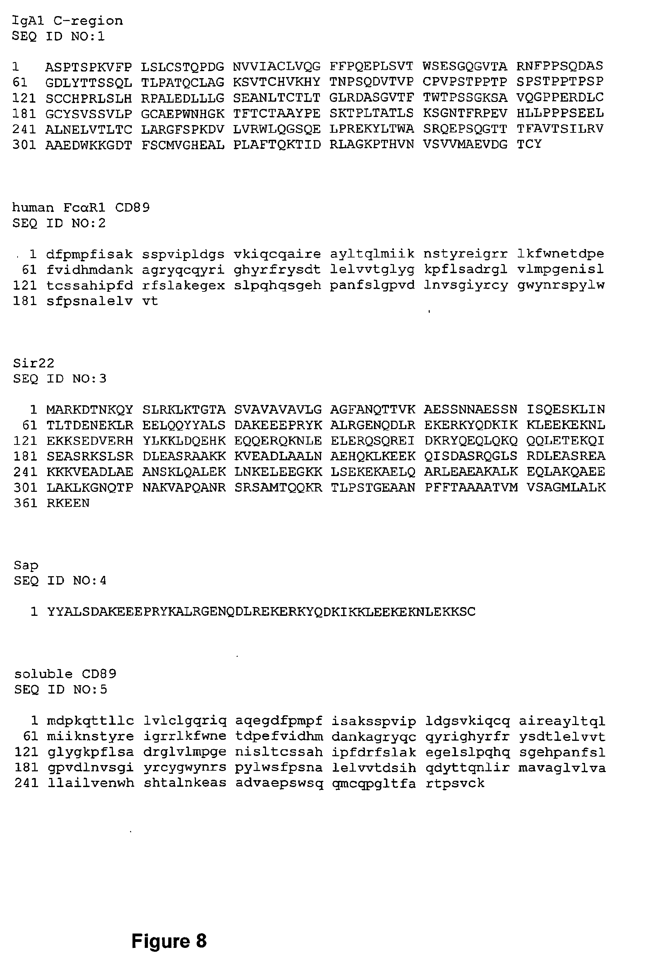

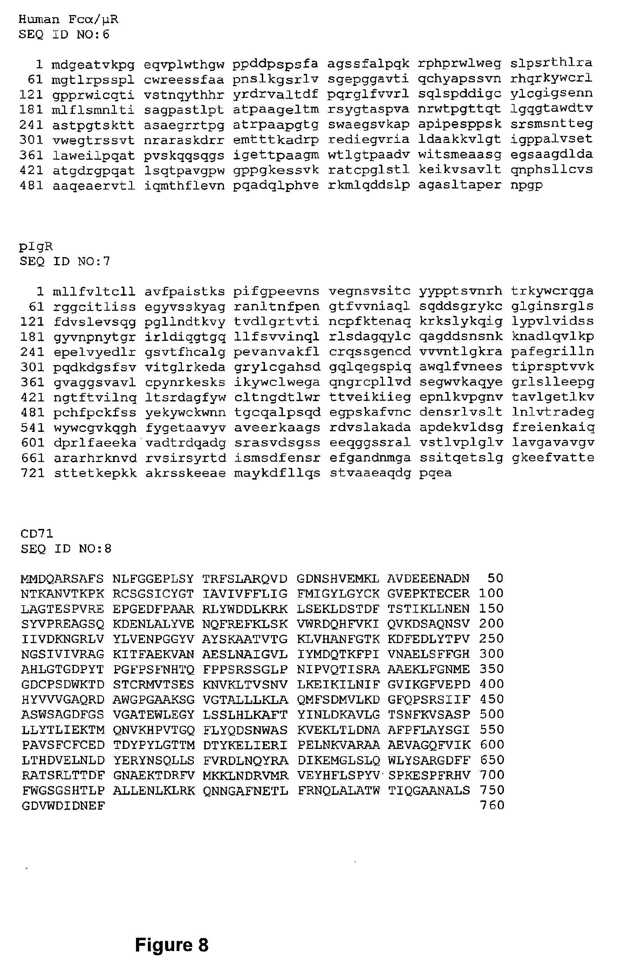

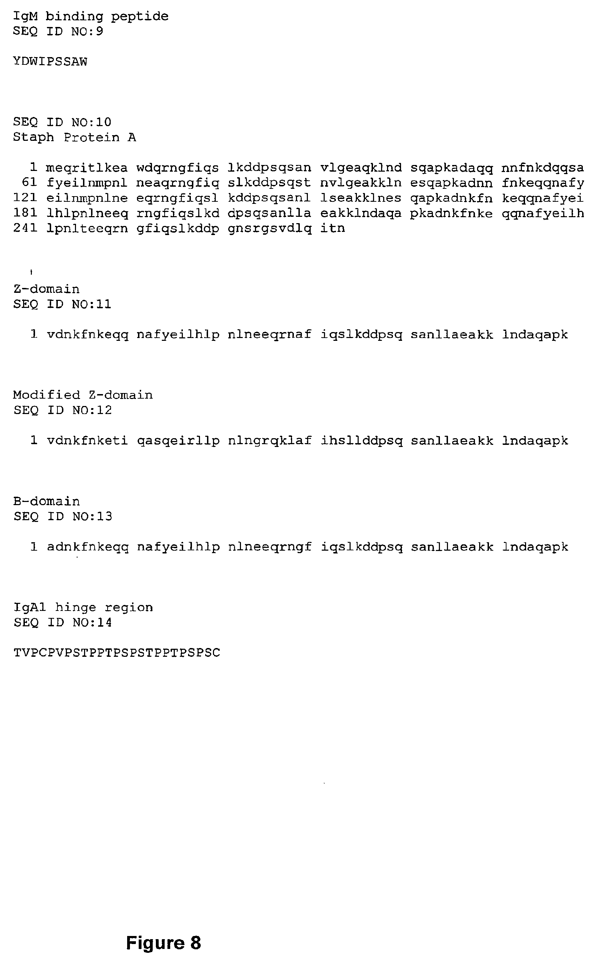

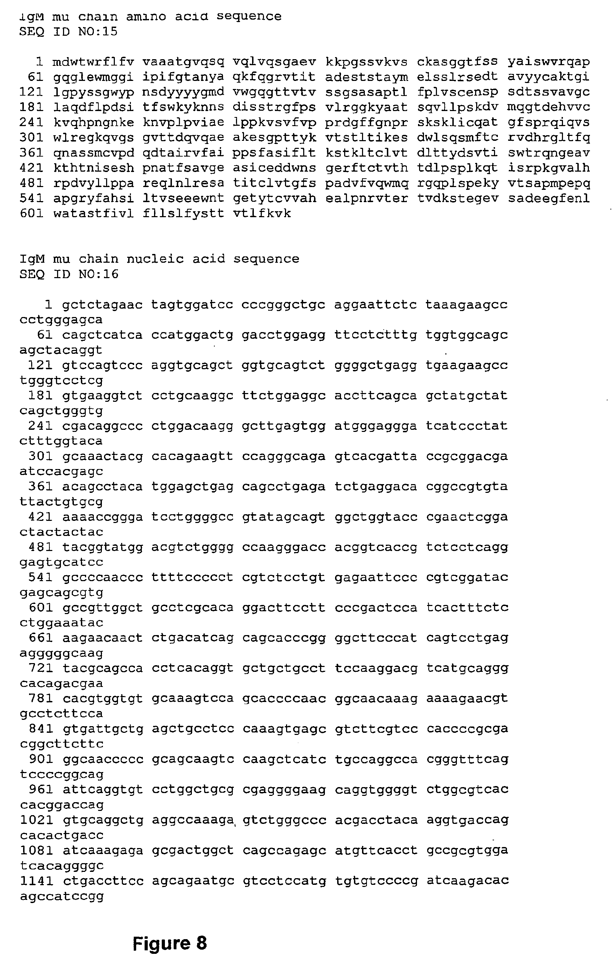

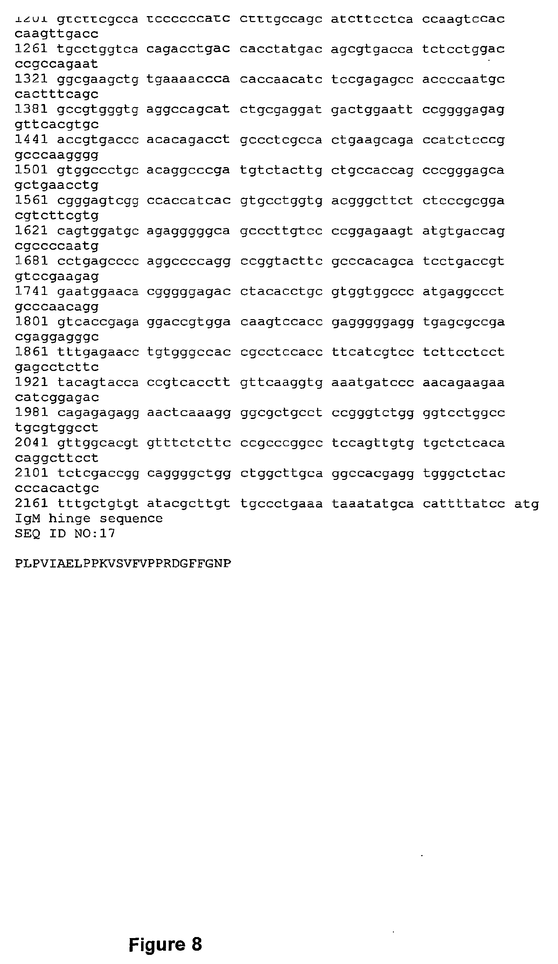

**Figure 8** is a list of amino acid and nucleic acid sequences including IgA1 C-region (SEQ ID NO:1), CD89 (SEQ ID NO:2), Sir22 (SEQ ID NO:3), Sap (SEQ ID NO:4), soluble CD89 (SEQ ID NO:5), Human Fcα/µR (SEQ ID NO:6), pIgR (SEQ ID NO:7), CD71 (SEQ ID NO:8), IgM binding peptide (SEQ ID NO:9), Staph Protein A (SEQ ID NO:10), Z-domain (SEQ ID NO:11), modified Z-domain (SEQ ID NO:12), B-domain (SEQ ID NO:13), the IgA1 hinge region (SEQ ID NO:14), IgM mu chain amino acid sequence (SEQ ID NO:15), IgM mu chain nucleic acid sequence (SEQ ID NO:16), and the IgM hinge sequence (SEQ ID NO: 17).

### Detailed Description

The present invention uses radiologic scans to identify patients who have glomerular-based renal diseases, such as IgA nephropathy (IgAN), Henoch-Schönlein purpura (HSP), and IgM nephropathy (IgMN). IgAN is characterized by a time-dependent (years) deposition of IgA1 immunoglobulins (SEQ ID NO: 1) into the glomeruli of the kidney cortex. HSP is closely related to IgAN, with the same pattern of IgA1 deposition and kidney injury. IgMN is characterized by IgM deposition in the mesangium of glomeruli. To identify those patients with renal disease who have IgA1 or IgM deposition, it has been necessary to carry out a closed needle biopsy of the kidney, a somewhat painful procedure of moderate risk that yields a tissue core for pathological examination. With the present invention IgA1 or IgM deposition can be diagnosed using radiologic imaging methods in which the IgA or IgM deposits in the kidney cortex are detected using an injected, labeled IgA-binding or IgM-binding molecule.

When one biopsies the kidney in order to make the diagnosis of kidney disease, the key criteria for diagnosis of IgAN, HSP, and IgMN is the presence of dominant IgA1 or IgM deposits in the kidney glomeruli, the only site of IgA or IgM deposition. As normal persons and those with non-IgAN, non-IgM kidney diseases do not have significant amounts of IgA1 or IgM in their renal glomeruli, the present invention represents a macro-scale, non-invasive detection method for IgA or IgM in the kidney cortex. Another advantage of the present invention is that scanning provides information about the entire kidney cortices in both kidneys, thus reducing sampling error inherent in needle biopsy methods (Sund et al., Nephrol. Dial. Transplant. 14:2445-2454, 1999).

The invention features several IgA-specific peptides that target IgA1, available radioisotopes by which they may be labeled, and linkers used for this labeling. In one embodiment, radionuclide-labeled IgA-binding peptides or proteins are used as diagnostic radiopharmaceuticals to detect IgA deposits in the kidney. The injected radiolabeled IgA-binding peptides bind to IgA throughout the body. Because there is little IgA deposited in the glomeruli of a healthy kidney, IgA deposits in the glomeruli of patients with IgAN result in a high concentration of the emission of radiation from the cortex of the kidney, where the glomeruli are located. This radiation can be detected by various nuclear imaging techniques and therefore may be used to diagnose IgAN. For diagnosis of the kidney disease, antibodies of human origin, or antibodies that are humanized, or animal monoclonal antibodies with specificity for human IgA1 are well-suited as IgA binding compounds, and can also be used to detect IgA1 deposits in the glomeruli.

Additional types of nephropathies including IgM nephropathy can be diagnosed similarly. Replacing an IgA-binding protein or antibody with an anti-human IgM antibody or its fragment, for example, IgM can be detected in IgM nephropathy.

### IgA Structure

Human immunoglobulin A (IgA) synthesis exceeds the combined total of all the other immunoglobulin classes (Rifai et al., J. Exp. Med. 191:2171-2181, 2000). It is estimated that 66 mg of IgA/kg of body weight is produced every day, compared with 34 mg of IgG and 7.9 mg of IgM. There are two isotypes of IgA, IgA1 (SEQ ID NO: 1) and IgA2. On mucosal surfaces (e.g., gut, respiratory tract, genital tract) both IgA1 and IgA2 are present, synthesized by local B cells. In the blood, however, IgA1 predominates, produced by B cells in the bone marrow, lymph nodes, and spleen (Donadio and Grande, N. Engl. J. Med. 347:738-748, 2002).

The main difference between the IgA1 (SEQ ID NO:1) and IgA2 subclasses is a 13 amino acid deletion in the IgA2 hinge region. This segment in IgA1 contains several serine and threonine amino acid residues that are O-glycosylated. The absence of this region in IgA2 explains the lack of O-linked oligosaccharides on IgA2 (Figure 1).

### IgM Structure

IgM (SEQ ID NO:15) can be found as a monomer on the surface of the B lymphocyte, but in circulation it exists mainly as a pentamer after being secreted from plasma cells. The IgM pentamer has a molecular mass of ~850-1,000 kDa while each monomer is ~180 kDa. IgM represents ~10% of total serum Ig and is the first isotype of antibody synthesized during a primary humoral response.

The normal plasma IgM level in adults is about 1 mg/ml with a half-life about 5 days. This compares with IgG level of 12 mg/ml with 25 days of half-life and IgA of 1.8 mg/ml with half-life of 6 days.

Carbohydrates constitute about 12% of the IgM protein by weight. The mu heavy chain of IgM consists of 4 CH domains. (Only mu and epsilon (IgE) heavy chains each have four constant heavy region domains---CH1, CH2, CH3 & CH4, while gamma (IgG), alpha (IgA), and delta (IgD) each have 3 constant heavy region domains.)

Due to the presence of 10 identical antigen-binding sites on a pentamer, IgM is an excellent agglutinating antibody. In addition, IgM is also efficient at activating complement. The IgM monomers are joined together as a pentamer through interchain disulfide bonds, and also by J chain, a 15 kDa peptide that attaches to two monomers of IgM and places the pentamer into a closed, apparently circular, conformation. (J chain also serves to link monomers of IgA together, forming dimers.)

### Diagnostic Compositions

The components of the diagnostic radiopharmaceutical for use herein include (1) a compound, peptide, or protein that specifically binds human IgA or IgM and (2) a compound (e.g., a radioisotope) capable of detection by a radiologic imaging technique (e.g., SPECT) chelated to the compound, peptide, or protein in a pharmaceutically acceptable carrier. Preferable agents include a bifunctional chelating agent (BFCA), which is used to chelate (1) and (2).

While any IgA-binding or IgM-binding compound, protein, or peptide may be used as a biomolecule for diagnosis, preferred candidates meet several or all of the following criteria: high specificity and affinity for human IgA or IgM; for IgA-mediated disorders, ability to differentiate IgA1 from IgA2; small molecules, as these are less immunogenic; a protein of human origin; easily expressed or chemically synthesized; easy to produce and modify; and appropriately glycosylated, containing galactose for asialoglycloprotein receptor (ASGPR) clearance thereby minimizing renal tubule retention.

### IgA- and IgM-binding Compounds/Peptides

IgA- and IgM-binding proteins or peptides can include native human IgA-binding receptors on cells; receptors that recognize both IgA and IgM; bacterial IgA-binding proteins and their derived peptides; anti-human IgA or IgM antibodies and their smaller derivatives (e.g., Fab); and IgA- and IgM-specific peptides discovered using methods such as phage display.

### IgA-specific Receptors

The natural functions of IgA-specific receptors include recruitment of inflammatory cells and mediators to inflammatory sites (CD89; SEQ ID NO:2), and the distribution of IgA, e.g., entry of IgA into secretions (pIgR; SEQ ID NO:7).

Human FcαR1 (CD89; SEQ ID NO:2; see Figure 2) is a membrane glycoprotein that contains two extracellular Ig-like domains (206 aa), a membrane-spanning region (19 aa), and a cytoplasmic tail (31 aa). At the transmembrane region, a positively charged arginine residue is necessary for association of CD89 with the FcR γ-chain. Like other Fc receptors lacking an intracellular signaling motif, FcαR's signaling into the cell is initiated via its association with FcR γ-chain. The FcR γ-chain is a homodimer-signaling unit with a size of 10 kDa. Binding to CD89 leads to phosphorylation of the intracellular, immunoreceptor tyrosine-based activation motif (ITAM) on the γ-chain, activating the signaling pathways downstream into the cell. FcαR1 binds both the monomeric and dimeric forms of IgA1 and IgA2. Transfection studies in leukocytes show that the FcαR1 does not bind IgG. FcαR1 is expressed only by myeloid cells, including neutrophils, monocytes, macrophages, and eosinophils. It was proposed that FcαR1 plays a role in the removal of IgA-antigen complexes from the circulation (Mattu et al., J. Bio. Chem. 273:2260-2272, 1998; Leung et al., J. Am. Soc. Nephrol. 11:241-249, 2000; Westerhuis, Pathogenetic Aspects of IgA-Nephropathy 2001*,* Chapter 1, Section IV: IgA receptors and IgAN, 2001, PrintParters, Ipskamp, Enschede).

A 206 amino acid soluble portion of recombinant CD89 (SEQ ID NO:5) has been successfully expressed in several research labs, and such a soluble receptor has the potential to be used as an IgA-detecting peptide for diagnosis of IgAN. Despite some controversial reports that soluble CD89 might exacerbate IgAN (Pierre Launay et al., J. Exp. Med. 191:1999-2009), this fragment is preferred for binding to IgA due to its high specificity and affinity. This protein is glycosylated, which likely accelerates its clearance via the asialoglycoprotein receptor (ASGPR). This minimizes background noise of unbound ligand in circulation and likely minimizes the clearance of radioactive nuclides through the renal degradation system, which also reduces background noise. More preferably, a smaller fragment of this peptide that retains IgA-binding activity is used.

### Receptors Specifically Binding Both IgA and IgM

The human Fcα/µ receptor (Fcα/µ R; SEQ ID NO:6) binds both IgA and IgM with intermediate to high affinity. Fcα/µ R is constitutively expressed on the majority of B-lymphocytes and macrophages (Sakamoto et al., Eur. J. Immunol. 31:1310-1316, 2001; Shibuya et al., Nat. Immunol. 1:441-446, 2000).

The polymeric Ig receptor (pIgR; SEQ ID NO:7) is an integral membrane component localized on the basolateral surface of secretory epithelial cells. It mediates the trans-epithelial transport of polymeric Ig, mainly dimeric IgA and pentameric IgM. pIgR is on most human secretory epithelia, including intestine, bronchus, salivary glands, renal tubule, and uterus, and it binds to J chains on polymeric immunoglobulins. Binding of IgA results in the protein being transferred from the lamina propria of the mucosa through the epithelial cell to the gut (or other IgA secretory sites) to reach the cell-free fluids bathing the mucus membranes. Secretory IgA (sIgA) is responsible for neutralization of microbes and toxins and prevents unwanted antigens from passing through the mucosal barrier (Mattu et al., J. Bio. Chem. 273:2260-2272, 1998; Leung et al., J. Am. Soc. Nephrol. 11:241-249, 2000).

Recently, other IgA receptors have been proposed, including the transferrin receptor (CD71; SEQ ID NO:8) expressed on mesangial cells (Haddad et al., J. Am. Soc. Nephrol. 14:327-337, 2003). Although the role of this protein in IgA1 deposition diseases is unknown, it may also be used in the present invention.

### Bacterial IgA-binding Peptides

Bacterial surface proteins that bind human IgA-Fc have been described in both group A streptococci (*Streptococcuspyogenes*) and group B streptococci (*Streptococcus agalactiae*; Sandin et al., J. Immunol. 169:1357-1364, 2002; Pleass et al., J. Biol. Chem. 276:8197-8204,2001; Johnsson et al., J. Biol. Chem. 274:14521-14524, 1999; Stenbere et al., J. Biol. Chem. 269:13458-13464, 1994). The IgA-binding proteins of *S*. *pyogenes* are members of the M protein family, a heterogeneous family of dimeric proteins that are important virulence factors. All M proteins bind one or more human plasma proteins, and about 50% of all *S. pyogenes* strains express an M protein that binds IgA-Fc. M proteins have structural features that foster IgA binding, for example, in several instances a heptad repeat pattern forms a coiled-coil dimer, which binds to specific sites on IgA. Protein Sir22 (also named protein M22, as it is among the M proteins; SEQ ID NO:3), which has been studied in detail by Sandin et al. (J. Immunol. 2002, 169:1357-1364), has such a structure with a stretch of 29 amino acids sufficient for IgA binding. A 50-residue synthetic peptide (SEQ ID NO:4) has been designed that includes the 29-residue IgA-binding region and which specifically binds to human IgA. This 50-mer, designated Streptococcal IgA-binding peptide or Sap (SEQ ID NO:4), has the properties of an isolated IgA-binding domain, and its binding site on IgA is known to be in the Fc region, the same site that binds human CD89. Sap is a homologue of amino acids 35-83 of Sir22 and was designed to include a C-terminal cysteine residue not present in Sir22. The cysteine was introduced to bring about dimerization of the Sap peptide, a process that can be enhanced by incubation with CuCl₂. The IgA-binding tests conducted by Lindahl et al. indicate that Sap dimerization is essential for IgA binding. Sap peptide immobilized on a solid support has been shown to deplete all isotypes, monomers and polymers of IgA from human serum, and eluates from Sap chromatographic columns contain only IgA. Thus, dimerized Sap represents a preferred IgA-binding peptide for IgAN diagnosis, having both IgA-binding specificity and apparent high affinity. Additionally, Sap can be synthesized by solid phase peptide synthesis (SPPS) methods, and a bifunctional chelating agent (BFCA; see below for details) can be added to either the N- or C-terminus while the residue is still in the solid phase on the resin, using methods standard in the art. Sap analogs are small (about 5.5 kDa for a monomer and 11 kDa when dimerized), thus minimizing antigenicity.

In *Staphylococcus aureus* protein A (SPA; SEQ ID NO:10), one can find five Ig binding domains. SPA binds strongly to the Fc region of immunoglobulins. Z-domain (SEQ ID NO:11) is a 58-aa residue recombinant protein derived from one of these five homologous domains (the B domain; SEQ ID NO: 13) in SPA. Z-domain was originally developed by changing a few amino acids of the B domain to enhance the stability of the latter as a gene-fusion partner for affinity purification of recombinant proteins by using IgG-containing resins. Using phage display technology, researchers at Karolinska Institute in Sweden modified the IgG-binding Z-domain into an IgA-binding peptide designated as Affibody_{IgA1} or modified Z-domain (SEQ ID NO: 12). (Graille et al., Proc. Natl. Acad. Sci. U.S.A. 97:5399-5404, 2000; Wahlberg et al., Proc. Natl. Acad. Sci. U.S.A. 100:3185-3190,2003; Rönnmark et al., Eur. J. Biochem. 269: 2647-2655, 2002; Braisted and Wells, Proc. Natl. Acad. Sci. U.S.A. 93:5688-5692, 1996). This peptide binds both human IgA1 and IgA2 with high specificity and affinity. The original IgG binding affinity was completely lost with these modifications. The binding site on IgA is believed to be in the Fc region. Given its binding to human IgA, this peptide is also preferred for IgAN diagnosis.

### Anti-human IgA or IgM Antibodies or Their Antigen-binding Fragments

Polyclonal or monoclonal anti-human IgA or IgM antibodies may also be used for the diagnosis of IgA or IgM kidney diseases. These antibodies may be derived from many sources including immunized animals or they may be prepared as animal/human chimeric proteins or humanized chimeric proteins, or human origin protein; all are strategies known in the art for making protein infusion therapies and/or diagnostic reagents. Both whole molecules and Ag-binding fragments of these antibodies can be used in the compositions used for the diagnostic methods of the invention. However, a preferred reagent is a monoclonal antibody of human origin, and, in the case of IgAN or HSP, preferably one that differentiates IgA1 from IgA2. Also, it is preferred that subunits of the antibody protein, which are preferred over the full-length antibody, maintain IgA- or IgM-recognition specificity (Reff et al., Canc. Control 9:152-166, 2002; Gorman and Clark, Semin. Immunol. 2:457-66, 1990; Antibodies as Medicines 2000 by Biotech Analytics).

In a preferred embodiment, small antibody fragments of anti-human IgA1 or anti-human IgM (e.g., human-originated fragments, humanized chimeric fragments, chimeric fragments, and animal origin fragments) are used. The fragments of the antibody that retain antigen-binding activity may be monovalent Fab, Fv, or scFv; or bivalent F(ab)'2 or diabodies. (See the schematic diagrams (Hudson and Souriau, Nat. Med. 9:129-134,2003) in the Figure 4 below). Preferably, the Fc region is deleted from the molecule.

In one particular example, the anti-human IgA1 antibody is directed at epitopes in the hinge region because, as described above, this region is unique to IgA1. Such human origin anti-human IgA1 hinge region Fab may be readily made by phage display technology through a large phage antibody library developed by commercial providers such as Cambridge Antibody Technology (Cambridge, UK). This technology has advantages over methods for raising conventional monoclonal antibodies in being rapid, and in allowing access to the gene for ease of modification.

In another example, anti-IgM monoclonal antibodies, raised using methods standard in the art (e.g., those described herein), are used to diagnose IgMN. These antibodies can be raised using the unique mu chain hinge sequences that bridge CH1 and CH2 domains of the constant region as a target antigen (PLPVIAELPPKVSVFVPPRDGFFGNP; SEQ ID NO:17). In addition, IgM-specific antibodies can be raised against isolated, intact Fc regions of the IgM protein, such Fc produced by trypsin cleavage at high temperature (Plaut and Tomasi, Proc. Natl. Acad. Sci. USA, 65:318-322,1970).

### IgM-binding Compounds

With the use of phage display technology, a peptide, YDWIPSSAW (SEQ ID NO:9), has been identified that binds with high affinity to murine IgM. This peptide also inhibits human rheumatoid factor (RF, mainly IgM class of anti-IgG autoimmune antibody) induced agglutination of IgG-coated latex beads, and shows no binding abilities to other immunoglobulins (Pati M. Glee et al. J Immunol, 1999, 163: 826-833).

### Identifying Novel IgA- or IgM-binding Compounds

The compositions used for the methods of the invention may also employ novel IgA-binding or IgM-binding compounds identified using techniques such as phage display. Phage display is a combinatorial screening technique, allowing the discovery and characterization of proteins that interact with a desired target by using multiple genes from a gene bank (George P. Smith, Science 228:1335, 1985). These genes represent a required diversity generated by DNA recombinant technology; therefore, each phage displays a unique random peptide. These genes are inserted into phages by replacing preexisting genes, thus creating a phage library. Premade libraries are readily available (e.g., Novagen T7Select) with accompanying kits. Novagen's T7 system is a lytic phage display that is preferred for cDNA libraries (J. Imm. Meth. 231:39; Nature Biotech. 19:1193), but other phages may also be used, such as non-lytic M13 bacterial filamentous phage, which is based on N-terminal fusion to surface coat proteins pIII and pVIII. The modified phages will then express the protein coded by the inserted DNA on its surface coat. In the case of M13, pIII display has a lower valency (1-5 per virion) and thus can be used to find high affinity compounds whereas pVIII has high valency (~200 per virion) and can be used to find very low affinity binding compounds. Phage display services are commercially available through companies including Dyax Corp. of Cambridge, Mass., which offers four phage libraries: human antibody, proteins, structured peptides, and linear peptides. The phages from an entire phage library are incubated with the targeted proteins, (e.g., the alpha chain of IgA or the mu chain of IgM). Phages that display peptides with high affinity and specificity for IgA or IgM are then selected. The polynucleotide sequences coding for these peptides are identified and sequenced, and additional peptides can be produced for further analysis. The highest affinity and specificity protein, along with other qualities such as immunogencity, are then chosen as the final candidate to be used in the compositions used for the methods of the invention. Phage display gene banks or library may include millions of related genes, and thus may be employed to identify IgA- or IgM-binding compounds useful for compositions used for the diagnostic methods of the invention.

### Polypeptide Expression

In general, polypeptides for use in the invention may be produced by any standard technique; for example, by transformation of a suitable host cell with all or part of a polypeptide-encoding polynucleotide molecule or fragment thereof in a suitable expression vehicle.

Those skilled in the field of molecular biology will understand that any of a wide variety of expression systems may be used to provide the recombinant polypeptide. The precise host cell used is not critical to the invention. A polypeptide for use in the invention may be produced in a prokaryotic host (e.g., *E. coli*) or in a eukaryotic host (e.g., *Saccharomyces cerevisiae,* insect cells, e.g., Sf21 cells, or mammalian cells, e.g., NIH 3T3, HeLa, or preferably COS cells). Such cells are available from a wide range of sources (e.g., the American Type Culture Collection, Rockland, Md.; also, see, e.g., Current Protocols in Molecular Biology, Eds. Ausubel et al., John Wiley and Sons). The method of transformation or transfection and the choice of expression vehicle will depend on the host system selected. Transformation and transfection methods are described, e.g., in Ausubel et al. (supra); expression vehicles may be chosen from those provided, e.g., in Cloning Vectors: A Laboratory Manual (Pouwels, P. H. et al., 1985, Supp. 1987).

One particular bacterial expression system for polypeptide production is the *E. coli* pET expression system (Novagen, Inc., Madison, Wis.). According to this expression system, DNA encoding a polypeptide is inserted into a pET vector in an orientation designed to allow expression. As the gene encoding such a polypeptide is under the control of the T7 regulatory signals, expression of the polypeptide is achieved by inducing the expression of T7 RNA polymerase in the host cell. This is typically achieved using host strains which express T7 RNA polymerase in response to IPTG induction. Once produced, recombinant polypeptide is then isolated according to standard methods known in the art, for example, those described herein.

Another bacterial expression system for polypeptide production is the pGEX expression system (Pharmacia). This system employs a GST gene fusion system which is designed for high-level expression of genes or gene fragments as fusion proteins with rapid purification and recovery of functional gene products. The polypeptide of interest is fused to the carboxy- terminus of the glutathione S-transferase protein from *Schistosoma japonicum* and is readily purified from bacterial lysates by affinity chromatography using Glutathione Sepharose 4B. Fusion proteins can be recovered under mild conditions by elution with glutathione. Cleavage of the glutathione S-transferase domain from the fusion protein is facilitated by the presence of recognition sites for site-specific proteases upstream of this domain. For example, polypeptides expressed in pGEX-2T plasmids may be cleaved with thrombin; those expressed in pGEX-3X may be cleaved with factor Xa.

Once the recombinant polypeptide is expressed, it is isolated, e.g., using affinity chromatography. In one example, an antibody (e.g., produced as described herein) raised against a polypeptide for use in the invention may be attached to a column and used to isolate the recombinant polypeptide. Lysis and fractionation of polypeptide-harboring cells prior to affinity chromatography may be performed by standard methods (see, e.g., Ausubel et al., supra).

Once isolated, the recombinant polypeptide can, if desired, be further purified, e.g., by high performance liquid chromatography (see, e.g., Fisher, Laboratory Techniques In Biochemistry And Molecular Biology, eds., Work and Burdon, Elsevier, 1980).

Polypeptides for use in the invention, particularly short peptide fragments, can also be produced by chemical synthesis (e.g., by the methods described in Solid Phrase Peptide Syntheses, 2nd ed., 1984 The Pierce Chemical Co., Rockford, Ill.).

These general techniques of polypeptide expression and purification can also be used to produce and isolate useful peptide fragments or analogs (described herein).

The short peptides such as 50-mer Streptococcal IgA-binding peptide (Sap) and 58-mer modified Z-domain can also be chemically synthesized.

### Antibody Production

To generate antibodies, any standard technique may also be used. For example, a coding sequence for IgA1, IgM, or fragments thereof (e.g., the hinge region of IgA1, amino acids 217-241) may be chemically synthesized or expressed as a C-terminal fusion with glutathione S-transferase (GST) (Smith and Johnson, Gene 67:31-40, 1988). The fusion protein is purified on glutathione-Sepharose beads, eluted with glutathione, cleaved with thrombin (at the engineered cleavage site), and purified to the degree necessary for immunization of mice or rabbits. Primary immunizations are carried out with Freund's complete adjuvant or a similar adjuvant and subsequent immunizations with Freund's incomplete adjuvant. Antibody titres are monitored by ELISA or Western blot and immunoprecipitation analyses using the thrombin-cleaved polypeptide fragment of the GST fusion protein. Immune sera are affinity purified using CNBr-Sepharose-coupled polypeptide. Antiserum specificity is determined using a panel of unrelated GST proteins.

As an alternate or adjunct immunogen to GST fusion proteins, peptides corresponding to relatively unique immunogenic regions of IgA or IgM may be generated and coupled to keyhole limpet hemocyanin (KLH) through an introduced C-terminal lysine. Antiserum to each of these peptides is similarly affinity purified on peptides conjugated to BSA, and specificity tested in ELISA and Western blots using peptide conjugates, and by Western blot and immunoprecipitation using the polypeptide expressed as a GST fusion protein.

Alternatively, monoclonal antibodies which specifically bind IgA or IgM are prepared according to standard hybridoma technology (see, e.g., Kohler et al., Nature 256:495, 1975; Kohler et al., Eur. J. Immunol. 6:511, 1976; Kohler et al., Eur. J. Immunol. 6:292, 1976; Hammerling et al., In Monoclonal Antibodies and T Cell Hybridomas, Elsevier, N.Y., 1981; Ausubel et al., supra). Once produced, monoclonal antibodies are also tested for specificity by Western blot or immunoprecipitation analysis (by the methods described in Ausubel et al., supra). Antibodies which specifically recognize IgA, especially the IgA1 isotype, or IgM are considered to be useful in the invention. Alternatively monoclonal antibodies may be prepared using IgA or IgM and a phage display library (Vaughan et al., Nat. Biotechnol. 14:309-314, 1996).

Preferably, antibodies are produced using fragments of IgA or IgM which lie outside generally conserved regions (e.g., the 13 amino acid region in IgA1, absent in IgA2; see Figure 1) or unique sequence in IgM hinge region; see SEQ ID NO:14 and appear likely to be antigenic by criteria such as high frequency of charged residues. In one specific example, such fragments are generated by standard techniques of PCR and cloned into the pGEX expression vector (Ausubel et al., supra). Fusion proteins are expressed in *E*. *coli* and purified using a glutathione agarose affinity matrix as described in Ausubel et al. (supra). To attempt to minimize the potential problems of low affinity or specificity of antisera, two or three such fusions are generated for each polypeptide, and each fusion is injected into at least two rabbits. Antisera are raised by serial injections, preferably including at least three booster injections.

Antibodies generated against IgAl may be employed to diagnose IgAN and HSP, and antibodies anti-IgM may be used to diagnose IgMN.

### Peptide Modifications

It may be desirable to modify peptides used in the compositions for use in the methods of the invention, as peptides that enter the circulation are rapidly degraded in plasma. Modifications are therefore desirable to prolong their half-life without reducing biological activity and binding specificity. For example, the tetradecapeptide somatostatin has only a 3 minute half-life in human plasma, far too short to be used as a radiopharmaceutical in cancer diagnosis. Thus, its analog, octreotide was developed to have a 90 minute half-life in the human circulation (Langer and Beck-Sickinger, Curr. Med. Chem. Anti-Canc. Agents 1:71-93, 2001). To resist degradation by exopeptidases, the peptides may be capped at the N- and/or C-terminus. This includes, for example, acetylation of the N-terminus, amidating or reducing the C-terminus, and N to C cyclization. These methods are standard in the art. To reduce susceptibility to endopeptidases the peptide may be modified by introducing D-amino acids, amino acid surrogates, or peptidomimetic sequences and spacers. Other ways to improve stability include modification of peptide bonds, replacement of amino-with imino- groups, methylation of amide nitrogens, and shortening of the natural amino acid sequence. Again, these methods are standard in the art.

### Radiometals

As indicated above, the diagnostic reagent further includes a detectable label, such as a radioactive label like a radioactive metal. Various radioactive metals are used in scintigraphy and their use varies with scintigraphy type, diagnosis being attempted, and cost and availability of the isotope. A key determinant in the isotope chosen is the scinitigraphy type, and radioactive decay needed. SPECT and PET use different isotopes because SPECT uses gamma emitters, whereas PET uses positron emitters. Isotopes can be produced in four different ways, which affects their cost. Those produced in a generator come from the conversion of a long half-life radioactive isotope to one of shorter half-life. This makes them practical for use even in small hospitals, and their cost is lower. Other radioactive metals are more expensive as they are produced in nuclear reactors, cyclotrons, and linear accelerators, and this may limit availability for smaller hospitals, and those in more remote areas. As for diagnosis, the radiometal used is in part dictated by the time needed to reach and to bind to the target. In the case of IgA-binding peptides, a longer half-life in the range of 4-80 hours may be preferred.

Metastable technetium-99m (^{99m}Tc) is used in 85% of scans, which is about 7 million each year in the U.S. alone. The properties of ^{99m}Tc are virtually ideal for diagnostic imaging; the γ-radiation of 140 keV falls within the ideal range of today's gamma cameras, and a half-life of 6 hours is long enough to synthesize the labeled radiopharmaceuticals, perform quality control, inject it into the patient, and carry out imaging. But the half-life is short enough to enable administration of sufficiently high doses with minimal patient risk, and at the concentration levels used (< 10⁻⁶ M), neither the resulting gamma radiation nor the soft beta decay of ⁹⁹Tc is hazardous. ^{99m}Tc is readily available at low cost from its parent nuclide ⁹⁹Mo (t_{1/2} 66 h), produced in a ⁹⁹Mo/^{99m}Tc generator.

Gamma-emitting Indium-111 (¹¹¹In) has been extensively used for labeling monoclonal antibodies and peptides. The first peptide radiopharmaceutical approved for clinical use by the FDA was the ¹¹¹In-labeled somatostatin analog OctreoScan^{®} (Mallinckrodt, St. Loius, Mo). The chemistry and half-life of this isotope (67.9 h) makes it ideal for labeling immunoglobulins, where imaging may be performed several days after the initial injection. ¹¹¹In is produced in a cyclotron from Cadmium-111, and thus it is less available in comparison to ^{99m}Tc. Ion exchange and solvent extraction are the methods commonly used to separate Indium-111 from parent cadmium. However, for the purpose of radiolabeling an IgA-binding peptide, the longer half-life of ¹¹¹In compared to ^{99m}Tc provides the IgA-binding peptide more time to reach IgA deposits in the kidney and provides more time for clearance of circulating tagged IgA.

Galium-67 (⁶⁷Ga) is produced in a cyclotron from zinc-68 (¹⁸Zn), and was the first nuclide produced for human use in 1953. The separation techniques may include solvent extraction and ion exchange, or both. The half-life of ⁶⁷Ga is over 78 hours, and its energy decay characteristics make it suitable for either gamma scintigraphy or PET imaging.

The radionuclides of copper offer a selection of diagnostic epitopes including ⁶⁰Cu, ⁶¹Cu, ⁶²Cu, and ⁶⁴Cu. The positron-emitting ^{6a}Cu is cyclotron produced. ⁶⁴Cu is preferred for labeling proteins, peptides, and agents with long blood clearance, which is likely an advantageous property of IgA-binding proteins. ⁶⁴Cu has a half-life of 12.7 hours.

Tables 1 and 2 below show the many gamma- and positron-emitting radiometals for diagnostic use, their decay characteristics, half-life, and methods of production (Anderson and Welch, Chem. Rev. 99:2219-2234, 1999). Any of these radiometals may be used in the methods and compositions described herein.

**Table 1. Gamma- and Beta-Emitting Radionuclides**

| isotope | hn (h) | production methods | decay mode | *E_{γ}* (keV) | *E_{β⁻}* (keV) |
|---|---|---|---|---|---|
| ⁶⁷Cu | 62.01 | accelerator, ⁶⁷Zn(n,p) | *β*⁻ (100%) | 91, 93, 185 | 577, 484, 395 |
| ⁶⁷Ga | 78.26 | cyclotron | EC (100%) | 91, 93, 185, 296 388 | |
| ⁹⁰Y | 64.06 | ⁹⁰Sr/⁹⁰Y generator | *β*⁻ (72%) | | 2288 |
| ¹¹¹In | 67.9 | cyclotron, ¹¹¹Cd(p,n)^{U1}In | EC (100%) | 245.172 | |
| ^{99m}Tc | 6.0 | ⁸⁹Mo/^{90m}Tc generator | IT (100%) | 141 | |
| ²⁰¹Tl | 72 h | cyclotron ²⁰³Tl(p,3n)²⁰¹Pb(p,n)²⁰¹Tl | EC (100%) Hg X-rays | 135, 167 | |

**Table 2. Positron-Emitting Radionuclides**

| Isotope | *t*_{1/2}(h) | methods of production | decay mode | *E*_{β}+ (keV) |
|---|---|---|---|---|
| ⁵⁵Co | 17.5 | cyclotron, ⁵⁴Fe(d,n)⁵⁵Co | β⁺ (77%) | 1513, 1037 |
| | | | EC (23%) | |
| ⁶⁰Cu | 0.4 | cyclotron, ⁶⁰Ni(p,n)⁶⁰Cu | β⁺ (93%) | 3920, 3000 |
| | | | EC (7%) | 2000 |
| ⁶¹Cu | 3.3 | cyclotron, ⁶¹Ni(p,n)⁶¹Cu | β⁺ (62%) | 1220, 1150 |
| | | | EC (38%) | 940, 560 |
| ⁶²Cu | 0.16 | ⁶²Zn/⁶²Cu generator | β⁺ (98%) | 2910 |
| | | | EC (2%) | |
| ⁶⁴Cu | 12.7 | cyclotron, ⁶⁴Ni(p,n)⁶⁴Cu | β⁺ (19%) | 656 |
| | | | EC (41%) | |
| | | | β⁻ (40%) | |
| ⁶⁵Ga | 9.5 | cyclotron, ⁶³Gu(α,nγ)⁶⁵Ga | β⁺ (56%) | 4150, 935 |
| | | | EC (44%) | |
| ⁶⁸Ga | 1.1 | ⁶⁸Ge/⁶⁸Ga generator | β⁺ (90%) | |
| | | | EC (10%) | 1880, 770 |
| ⁸²Rb | 0.022 | ⁸²Sr/⁸²Rb generator | β⁺ (96%) | 3150 |
| | | | EC (4%) | |
| ⁸⁵Y | 14.7 | cyclotron, ⁸⁶Sr(p,n)⁸⁶Y | β⁺ (33%) | 2335, 2019 |
| | | | EC (66%) | 1603, 1248 1043 |

### BFCAs

In radiopharmaceuticals, bifunctional chelating agents may be used to bridge the biological molecules and radiometals. There are many BFCAs available, any of which may be used in the invention. In one particular example, BFCAs that bind ^{99m}Tc have been most widely used because ^{99m}Tc is the metal used in 85% of the diagnostic scans currently in clinical use (Langer and Beck-Sickinger, Curr. Med. Chem. Anti-Canc. Agents 1:71-93, 2001). These BFCAs form a complex that has thermodynamic stability, kinetic inertness with respect to dissociation, and ability to stabilize the oxidation state of Tc. A variety of BFCAs have been developed for ^{99m}Tc including triamdiethiols N₃S, diamide-dithiols N₂S₂, propyleneamineoxime (PnAO), and hydrazinonicotinic acid (HYNIC). Some structures are shown in (Figure 5). A newer approach employs an organometallic species [M(CO)₃(H_{2O})₃]⁺ (M = ⁹⁹Tc, ^{99m}Tc, ¹⁸⁵/¹⁸⁷Re, ^{186/188}Re), which is a Tc/Re(I) precursor for labelling pharmacophores. The cationic species, available after short reaction times in high radiochemical purity, offer several advantages including maintenance of the oxidation state, stability in serum, and formation of stable complexes with biological ligands. Histidine and PADA (picolylamine-N,N-diacetic acid; Figure 6) have been identified as suitable BFCAs. Histidine has been used to radiolabel a neurotensin derivative, and PADA for the labelling of bombesin- and neuropeptide Y derivatives with ^{99m}Tc.

The BFCA of choice for Indium-111 is diethylene-triaminepentaacidic acid (DTPA), and a variety of peptides have been labeled with DTPA (Figure 7). The second most widely used chelator for ¹¹¹In is DOTA (tetraazacyclododecanetetraacidic acid) (Figure 7), which also can be used for complexing the therapeutic radionuclide Yttrium-90 (⁹⁰Y).

Tripeptides and tetrapeptides such as Lys-Gly-Cys, Cys-Gly-Cys, Gly-Gly-Cys, and Gly-Ala-Gly-Gly can also be used as BFCAs (Langer and Beck-Sickinger, Curr. Med. Chem. Anti-Canc. Agents 1:71-93, 2001). For example, ^{99m}Tc has been used to label vasoactive intestinal peptide (VIP), and its analog TP3654, which carries the chelating amino acid sequence GAGG on the C-terminus of VIP (Thakur et al., J. Nucl. Med., 41:107,2000). In another report, ^{99m}Tc-labelling via N-terminal Ac-Cys-Gly-Cys-Gly (CGCG) chelation to α-melanocyte-stimulating hormone (MSH) resulted in promising candidates for peptide-based radio-diagnosis (Chen et al., Nucl. Med. Biol. 26:687-693, 1999).

### Non Radioactive Labels

In addition to radiolabeled compounds, paramagnetic substances and quantum dot (qdot) technology can also be used in the compositions for use in the methods of the invention. Coupling paramagnetic substances to an IgA-binding or IgM-binding compound allows imaging via MRI, and qdot allows fluorescent imaging.

### Paramagnetic Substances

If an imaging technique (e.g., MRI) that does not require radioactivity is used in the present invention, a nonradioactive label may be substituted for the radiometal described above. For example, a paramagnetic substance (e.g., gadolinium) can be conjugated to an IgA-binding protein using methods standard in the art. When the compound is injected into a mammal and imaged by MRI, there is a difference in magnetic properties where the paramagnetic substances settle down. These differences can be used to identify IgA or IgM deposition in the glomeruli of the kidneys, and thus to diagnose an IgA or IgM kidney disease.

### Quantum Dots

Quantum dots (qdots) are fluorescent semiconductor nanocrystals made from variety of materials, for example CdS, CdSe, CdTe, CdHgTe/ZnS, InP, InAs, and PbSe. For nanocrystals smaller than the so-called Bohr exciton radius (a few nanometers), energy levels are quantized, with values directly related to the crystal size (an effect called quantum confinement). Thus, they have some distinguishing characteristics from commonly used fluorophores. One advantage of the qdots is that their size and shape can be precisely controlled by the duration, temperature, and ligand molecules used in the synthesis. The qdots' absorption and emission properties can also be controlled, because they are composition- and size-dependent.

The core of qdots may be wrapped by polymer coating materials to make them soluble in aqueous solutions and to prevent the release of cytotoxic Cd²⁺ or Se²⁻ ions from the qdot core. To add function to qdots, the outermost layer can include conjugated compounds (e.g., antibodies and bioactive peptides) that impart specific functionalities (e.g., IgA or IgM binding). If reduction of accumulation in liver and bone marrow is desired, high molecular weight polyethylene glycol (PEG) molecules can be added to the coating.

In one embodiment, qdots may be used alone for imaging purposes. Qdots tagged with antibodies (e.g., anti-IgA or anti-IgM antibodies) or binding compounds (e.g., IgA- or IgM-binding compounds) may be used to target the qdots to specific molecules. Such qdots can be injected into a mammal, and imaged using fluorescent techniques as described by Gao et al. (Nat. Biotech. 22:969-976, 2004).

In another embodiment, qdots may be coupled to radiolabeled compounds (e.g., the radiometals described herein) or paramagnetic substances (e.g., gadolinium). These qdots can be injected into a mammal, and visualized using an appropriate imaging technique (e.g., MRI, SPECT, PET, or planar scan).

### Formulation of Diagnostic Compositions

The compound(s) for use in the methods of the invention may be contained in any appropriate amount in any suitable carrier substance, and is generally present in an amount of 1-95% by weight of the total weight of the composition. The composition is preferably provided in a dosage form that is suitable for parenteral (e.g., intravenous) administration route. The diagnostic compositions may be formulated according to conventional pharmaceutical practice (see, e.g., Remington, The Science and Practice of Pharmacy (20th ed.), ed. A.R. Gennaro, Lippincott Williams & Wilkins, 2000 and Encyclopedia of Pharmaceutical Technology, eds. J. Swarbrick and J. C. Boylan, 1988-1999, Marcel Dekker, New York).

### Parenteral Compositions

The diagnostic compositions may be administered parenterally by injection, infusion, or implantation (intravenous or the like) in dosage forms, formulations, or via suitable delivery devices or implants containing conventional, non-toxic pharmaceutically acceptable carriers and adjuvants. The formulation and preparation of such compositions are well known to those skilled in the art of pharmaceutical formulation.

The composition may be in form of a solution, a suspension, an emulsion, an infusion device, or a delivery device for implantation, or it may be presented as a dry powder to be reconstituted with water or another suitable vehicle before use. Apart from the diagnostic compound(s), the composition may include suitable parenterally acceptable carriers and/or excipients. Furthermore, the composition may include suspending, solubilizing, stabilizing, pH-adjusting agents, tonicity adjusting agents, and/or dispersing agents.

As indicated above, the diagnostic compositions may be in a form suitable for sterile injection. To prepare such a composition, the diagnostic compound(s) are dissolved or suspended in a parenterally acceptable liquid vehicle. Among acceptable vehicles and solvents that may be employed are water, water adjusted to a suitable pH by addition of an appropriate amount of hydrochloric acid, sodium hydroxide or a suitable buffer, 1,3-butanediol, Ringer's solution, dextrose solution, and isotonic sodium chloride solution. The aqueous formulation may also contain one or more preservatives (e.g., methyl, ethyl or n-propyl p-hydroxybenzoate). In cases where one of the compounds is only sparingly or slightly soluble in water, a dissolution enhancing or solubilizing agent can be added, or the solvent may include 10-60% w/w of propylene glycol or the like.

### Dosages of Compounds

While the attending physician ultimately will decide the appropriate amount of the compound or compounds for diagnosis of an IgA or IgM kidney disease, typically 1, 2, 3, 4, 5, 6, 7, 8, 9, or 10 mCi of a radiolabeled compound will be administered intravenously in a pharmaceutically acceptable carrier. Dosages are determined based on several considerations, including the dosage required for imaging technique (e.g., SPECT) used and by the clearance and absorption characteristics of the compound. Radioactive dosimetry can be used to ensure the maximum radiation allowed to each organ is not exceeded, based on published values.

### Imaging Techniques

Any standard imaging technique can be used, with scintigraphy being the preferred method. Those skilled in the art will know which types of scanner are used with specific radiographic agents.

### Scintigraphy

Scintigraphy involves the use of radioactive isotopes to diagnose and treat various diseases. It has applications in neurology, cardiology, oncology, endocrinology, lymphatics, urinary function, gastroenterology, pulmonology, and other areas. Generally, the radioactive isotope is chemically bonded to a specific compound, and then injected intravenously. Different radioactive isotopes are used for different scintigraphy methods and three common methods are SPECT, PET, and planar scan (Anderson and Welch, Chem. Rev. 99:2219-2234, 1999; Langer and Beck-Sickinger, Curr. Med. Chem. Anti-Canc. Agents 1:71-93,2001).

SPECT (Single Photon Emission Computer Tomography) is a nuclear imaging technique that uses gamma rays. After injection of an isotope, a rotating gamma camera collects images 360 degrees around a patient, selecting only photons of certain energy. The images from various levels of the body are processed and recombined to form a 3D image.

PET (Positron Emission Tomography) is a nuclear imaging technique that uses radioactive isotopes that decay by positron emission, resulting in the release of two photons in opposite directions. A 360-degree scanner detects these photons, but only paired photons are processed. The safety of PET scans is higher than body CT, with radiation absorbed from a PET scan averaging 5 mSv, whereas the radiation absorbed from a CT scan of the body ranges from 6 - 16 mSv.

Planar scanners were among the first generation of scintillation detecting devices and are currently used in many diagnostic procedures. They are in common use and yield two-dimensional images.

### MRI

In addition to scintigraphy methods, magnetic resonance imaging (MRI) can also be used. MRI uses strong magnetic fields to generate three dimensional tomographic images of patients' bodies and does not require radioactivity.

### Radiological Optimization

To further optimize the diagnostic methods, renal tubule retention may be reduced and pretargeting strategies employed.

### Renal Tubule Detention of Radioactive Chelates

Radiolabeled peptides, proteins, or their fragments smaller than approximately 60 kDa are filtered at the glomerulus and enter the renal tubule (Langer and Beck-Sickinger, Curr. Med. Chem. Anti-Canc. Agents 1:71-93, 2001; Thakur et al., J. Nucl. Med., 41:107, 2000). Under physiological conditions, the cells of the proximal tubule quantitatively reabsorb these peptides, which are then subject to lysosomal degradation. This reabsorption traps the radiometal chelate in the cell, resulting in high retention of the radiolabel in the renal medulla and reducing the scintigraphic sensitivity for detection of specific higher emissions from the medullary region of the kidney. However, in IgAN, IgA deposits are in the glomeruli located in the renal cortex, minimizing the problem of renal radiometal retention and the associated interference. Reduction of renal tubule retention in diagnosing an IgA or IgM kidney disease may still be desirable, and can be accomplished by using more lipophilic molecules. These molecules have a high content of lipophilic amino acids in the peptide, or have the addition of a fatty acyl moiety on the molecule. In one example, such lipophilic modification can be seen in RC-160, a somatostatin analog with increased lipophilicty that has fewer tendencies towards renal excretion (P. Dasgupta and R. Mukherjee, Br J Pharmacol (2000) 129, 101-109). A second example is stearyl-Nle¹⁷-VIP, a lipophilic analogue of 28-mer vasoactive intestinal peptide, VIP. (Gozes et al., J. Pharmacol. Exper. Therap. 273 (1995) 161-167). The lipophilic modification may promote metabolic degradation through the liver, rather than the kidney. Another way to reduce renal tubular accumulation of radiometals is to increase the size of the radiolabeled reagent to be large enough to avoid filtration through glomeruli (Langer and Beck-Sickinger, Curr. Med. Chem. Anti-Canc. Agents 1:71-93, 2001). A third method is to administer oral or injected basic amino acids (e.g., lysine) or their derivatives following administration of the diagnostic agent, thereby inhibiting renal tubular cell uptake of proteins and peptides and inducing a transient proteinuria (Behr et al., Eur. J. Nucl. Med. 25:201-212, 1998).

### Pretargeting Strategies

Pretargeting delivery is commonly used in tumor radioimmunoscintigraphy (RIS) and radioimmunotherapy (RIT) (Anderson and Welch, Chem. Rev. 99:2219-2234, 1999; Langer and Beck-Sickinger, Curr. Med. Chem. Anti-Canc. Agents 1:71-93, 2001; Chang et al., Mol. Canc. Ther. 1:553-563, 2002; Rossi et al., Clin. Canc. Res. 9:3886s-3896s, 2003). Pretargeting reduces or clears unbound radiolabeled molecules in the circulation, at the time when bound concentration is highest. The most widely used system is the biotin-streptavidin (SA) pair. Generally, the non-radiolabeled mAb-SA is injected first. As the clearance of antibodies is slow in circulation, unbound antibodies need to be cleared before delivering radiolabeled biotin. Thus, after 1-2 days of the first injection, a galactosylated clearing agent that interacts with mAb-SA is injected. This agent quickly clears away any circulating mAb-SA, but it is unable to clear bound mAb-SA. After another 1-10 hours, the radiolabeled biotin-BFCA is injected. Therefore, the radioactive chelate is only delivered to bound locations instead of to the circulating antibodies. Other possible strategies include a similar approach with a binding pair other than biotin-streptavidin or a system that requires only two steps, for example, as described below.

### Two Step Method

To reduce the intravascular IgA or IgM radioactivity background, a two step method may also be utilized. In this method, an anti-IgA1 antibody or an anti-IgM antibody is first prepared, from which an Fab domain is obtained by limited proteolysis. This Fab is then modified to include streptavidin (SA) and galactose (Gal). The streptavidin is added as a biotin ligand, and Gal is added to promote removal of circulating Fab-IgA1 or Fab-IgM complexes. As clearance of the SA-Gal-Fab by hepatic ASGPR (asialoglycoprotein receptors) is faster than binding to IgA or IgM, a suitable competitive inhibitor such as galactose-Ficoll, a synthesized polymer of galactose that effectively inhibits ASGPR function, is injected (Rifai et al., J. Exp. Med. 191:2171-2181, 2000). Simultaneously with or immediately following the galactose-Ficoll injection, the SA-Gal-Fab anti-IgA1 or SA-Gal-Fab anti-IgM, is injected into patients for pretargeting, binding to both circulating IgA1 and IgA1 deposited in the renal cortex, or circulating IgM and IgM deposited in the renal cortex. At 24-48 hours, when the effect of galactose-Ficoll ends, the circulating SA-Gal-Fab-IgA1 or SA-Gal-Fab-IgM complexes are cleared from the circulation via the hepatic ASGPR. However, the immobilized SA-Gal-Fab-IgA1 or SA-Gal-Fab-IgM complexes in the glomeruli persist, and are available for imaging.

Approximately one or two days after the first injection, with background vascular SA-Gal-Fab-IgA1 or SA-Gal-Fab-IgM complex substantially cleared, radiolabeled (^{99m}Tc or ¹¹¹In) biotin-HSA-Gal (biotinylated human serum albumin conjugated with galactose) is injected and binds to SA-Gal-Fab-IgA1 SA-Gal-Fab-IgM complexes in the kidney. After another 4-24 hours, the removal of unbound (free) radiolabeled Biotin-HSA-Gal from the circulation by hepatic ASGPR results in increased target to noise ratio in the renal cortex. In addition, albumin's size (66kDa) excludes the radioactive complex from being filtrated from glomeruli, thus reducing associated background radiation due to settling of radioactive chelates in the proximal tubule cells of the kidneys. Another advantage of this design is that streptavidin is a tetramer (MW: 4x13kDa=52kDa), binding 4 molecules of biotinylated ligands. This increases the imaging signal, with four moles of radioactive Biotin-HSA-Gal binding per mole streptavidin exposed in the renal cortex.

To carry out this method, anti-human IgA1 antibody or anti-human IgM antibody and its Fab fragment are produced to diagnose an IgA or IgM kidney disease. Mouse or chimeric monoclonal anti-human IgA1 hinge region or anti-human IgM antibodies are raised by conventional methods, for example, as described herein. The Fab fragment is then obtained by any standard technique, such as papain digestion, and then purified, for example, by gel-filtration chromatography followed by antigen-affinity chromatography.

To conjugate galactose molecules onto Fab, cyanomethyl-2,3,4,6-tetra-I-acetyl-1-thio-β-D-galactopyranoside (C-4141; Sigma) is dissolved in methanol at 0.1 M and mixed with 0.1 volume sodium methoxide (methanol sodium derivative; J.T. Baker Chemical Co., Phillipsburg, NJ). After 48 h at room temperature, this mixture is stored for weeks at 4°C. The conjugate is replaced in 0.25 M sodium borate buffer, pH 8.5, containing Fab at 1.0 mg/ml. After 2h at room temperature, the sample is dialyzed into phosphate-buffered saline. The antigen-binding function of the antibody is not altered by the conjugation (Ong et al., Canc. Res. 51:1619-1626, 1991).

Streptavidin (SA; MW:~52 kDa) binds specifically with biotin (244 Da). It is derived from the bacterium *Streptomyces avidinii* and bears similarity to chicken egg-white avidin both in three-dimensional structure and its ability to bind biotin with extremely high affinity (Kd=10⁻¹⁵ M). It is a tetrameric protein (4x13 kDa) capable of binding up to 4 biotin molecules. Unlike avidin, streptavidin is non-glycosylated and is essentially neutral in charge, whereas avidin (pI~10.5) is basic at neutral pH. Because of this, streptavidin has considerably less non-specific binding resulting in less background and has replaced avidin as the preferred reagent for applications where protein interactions may cause undesired background.

To conjugate SA to Gal-Fab, the Gal-Fab is chemically conjugated to SA (Genzyme, Cambridge, Mass) using succinimidyl 4-(*N*-maleimido-methyl) cyclohexane-1-carboxylate (SMCC). Excess SMCC is offered to SA in a 3:1 molar ratio in sodium borate at pH 8 containing 5% DMSO. After 30 min, the SMCC-SA is desalted by Sephadex G-25 (Amersham Pharmacia) gel filtration. The Gal-Fab is reduced with DTT, desalted by gel filtration and mixed in 1:1 molar ratio with SMCC-SA at 5 mg/ml total protein concentration in PBS. The reaction is monitored by size exclusion HPLC, and after sufficient conjugate has formed, *ca*. 50 min, the reaction is quenched by the addition of sodium tetrathionate to 5 mM to reoxidize unreacted thiols. The conjugate is separated from free SA by Q Sepharose chromatography. The conjugate is also separated from unconjugated Gal-Fab by affinity chromatography using immobilized iminobiotin (Pierce) equilibrated in 0.05 M sodium carbonate, 0.5 M sodium chloride, pH 11 and eluting with 0.05 M sodium acetate/0.5 M sodium chloride, pH 4. About 80% of the conjugate consists of SA/antibody in a 1:1 ratio, with the remainder primarily in a 2:1 ratio or higher (Axworthy et al., Proc. Natl. Acad. Sci. U.S.A. 97:1802-1807, 2000).

HSA (human serum albumin) is a single polypeptide chain protein containing 584 amino acids (66 kDa), and is the most abundant protein in the blood, accounting for about 60% of protein in the plasma. HSA is exclusively produced in liver, and unlike most other plasma proteins, it contains no carbohydrates. HSA has a half-life of about 19 days. The main functions of HSA include maintenance of colloidal osmotic pressure in the blood vessels, contribution to maintenance of acid/base metabolism, and transport of intrinsic substances and medications. Highly purified, virus free, recombinant human albumin in pharmaceutical excipient quality can be obtained commercially (e.g., GTC Biotherapeutics).

To prepare biotin-HSA-Gal, HSA is combined with 3-fold excess *N*-hydroxysuccinimide-LC-biotin (Pierce) in 0.5 M sodium borate (pH 8.5), 5% DMSO. After *ca.* 4 hr the solution is added to a 200-fold excess of freshly prepared neat 2-imino-2-methoxyethyl 1-thio-β-D-galactopyranoside. The mixture is stirred for 8 hr at room temperature and purified by diafiltration into PBS. The final material contains 1.6 moles of biotin per mole of HSA as determined by displacement of 2-(4'-hydoxyphenylazo)-benzoic acid (HABA) from SA and 30-40 moles of thiogalactose per mole of HSA as determined by a colorimetric anthrone assay (Axworthy et al., Proc. Natl. Acad. Sci. U.S.A. 97:1802-1807, 2000).

To conjugate bifunctional chelating agent (BFCA) DOTA (1,4,7,10-tetraazacyclododecane-1,4,7,10-tetraacetic acid) onto biotin-HSA-galactose, the COOH group of DOTA is activated by a carbodiimide reagent. Briefly, DOTA is dissolved in anhydrous DMSO at 80°C, and the solution is cooled under argon. A solution of *N*-hydroxy-2,5-pyrrolidinedione in DMSO is added dropwise to a stirred solution of DOTA, followed by the dropwise addition of *N*,*N*'-dicyclohexylcarbodiimide in DMSO. The molar ratio betweenDOTA:*N*-hydroxy-2,5-pyrrolidinedione:*N,N'*-dicyclohexylcarbodiimid e is 1:1.4:0.8. The reaction mixture is stirred overnight and then filtered to separate a by-product, dicyclohexylurea. The conjugation between DOTA and biotin-HSA-Gal is carried out at a molar ratio of 50:1 by adding an adequate volume of the DOTA-activated ester solution to the biotin-HSA-Gal dissolved in 0.1 M phosphate buffer (pH 8.0). After the overnight reaction, the conjugate is purified by a reverse-phase column in a FPLC system coupled with a UV detector and a radiodetector. A linear gradient method is applied using an aqueous 0.1 % trifluoroacetic acid solution (solvent A) and methanol (solvent B). The eluents are delivered at a flow of 4 ml/min, starting from 0% of solvent A to 100% of solvent B in 37 ml. Two peaks corresponding to the Biotin-HSA-Gal conjugates with DOTA are observed in the UV profile. The retention volume for biotin-HSA-gal conjugates with DOTA differ from that of unconjugated biotin-HSA-gal. An integrated fraction collector is used to recover each compound and further analysis by MALDI-TOF (matrix-assisted laser desorption/ionization combined with time-of-flight) mass spectrometry (Bussolati et al., Canc. Res. 61:4393-4397,2001) can be performed if desired.

To conjugate with the radiolabel, indium-111-chloride (¹¹¹InCl₃) with no carrier added is obtained from, for example, Mallinckrodt, Inc. (St. Louis, MO). DOTA-biotin-HSA-galactose conjugate is dissolved in 0.2 M ammonium acetate buffer (metal free; pH 7), and then incubated for 30 min at 100 C with ¹¹¹InCl₃ at a ratio of about 20 MBq ¹¹¹In/1 nmol conjugate. The purity of radiolabeled conjugate is assessed by instant thin layer chromatography (ITLC). The ITLC system uses an ITLC-SG (silica gel) support (Gelman Sciences, Ann Arbor, MI) and 4 mM EDTA (pH 4.0) as a mobile phase. The peptide-bound activity remains at the origin, and the previously uncomplexed radiometal moves at the solvent front as an EDTA complex. The radiolabeling efficiency is typically greater than 97% and is critically dependent on the chemical purity (metal cations) of the ¹¹¹InCl₃ (Smith-Jones et al., Endocrinology 140:5136-5148, 1999).

Galactose-Ficoll can be readily synthesized as follows. Cyanomethyl 1-thio-β-D-galactopyranoside is dissolved in methanol at an approximate ratio of 1.0 mmol per 10 ml, with the addition of 0.5 ml of 0.5 M sodium methoxide in methanol. After 48 hours of stirring at room temperature in a tightly capped glass vial, the methanol will be evaporated with a stream of nitrogen. To the dried residue is added 1 gram of Ficoll 70 or Ficoll 400 from Pharmacia or other manufacturers in 100 ml BBS (0.16 M sodium chloride-0.2 M sodium borate, pH 8.0). This is stirred at room temperature for 24 hours, then the reaction is stopped by adding 10 ml of 1.0 N acetic acid. The reaction mixture will be dialyzed exhaustively against distilled water (Plotz and Rifai, Biochemistry 1982,21, 301-308; Lee at el. Biochemistry 1976, 15, 3956-3963).

Administration of the compounds may be intravenous. In one example, 2.0 mCi of ¹¹¹In is injected into patients intravenously over a period of 30-60 seconds. Images are taken 24-48 hours post injection as described below; however, the time between administration and imaging may be altered as desired or based on clearance and retention characteristics of the particular compounds used in the diagnostic method.

Any of the several SPECT scanners commercially available may be used in this method (e.g., the GE MyoSIGHT, General Electric Company). One skilled in the art will know what settings (e.g., collimators, window size, and energies) can be used with specific radiometals or isotopes. In one example, a medium energy collimator is used for ¹¹¹In, with symmetric windows of 15-20% set at the 173 and 247 keV (the photopeaks of ¹¹¹In). In another example, a low energy collimator is used for ^{99m}Tc, with symmetric windows of 20% set at 140 keV. The number of images to be taken depends on the capabilities of the scanning system, and the desired image quality. A 64 x 64 matrix set of images may be taken, usually with the camera moving 360 degrees with 64 stops. If higher image quality is desired, a 128 x 128 or 256 x 256 matrix set of images may be taken. The scan may take 30-60 minutes during which time the patient must remain absolutely still. Additional patient preparation (e.g., oral hydration, cathartics, and enemas), if desired, may be performed to obtain optimal images.

The images can be used to determine the amount of radioactivity in the kidney of the patient. By comparing this amount of radioactivity to the radioactivity in the kidney of a patient known not to have IgA nephropathy or HSP, an increase in radioactivity detected in the kidney is diagnostic of IgA nephropathy or HSP.

Similarly, in the case where anti-human IgM is used in place of anti-human IgA, an increase in the amount of radioactivity determined from the images relative to the radioactivity in the kidney of a patient known not to have IgM nephropathy is diagnostic of IgM nephropathy.

## Claims

1. A compound which specifically binds IgA or IgM for use in *in vivo* diagnosing an IgA or IgM kidney disease in a mammal, preferably a human, the compound being linked to a first member of a binding pair and to galactose, wherein the compound is detected in the kidney of said mammal by a radiolabelled substance linked to a second member of the binding pair and to galactose, wherein detection occurs following clearance of unbound compound from circulation; and an increase in the amount of said compound in the kidney of said mammal relative to the amount in the kidney of a mammal without said kidney disease is diagnostic of said IgA or IgM kidney disease in said mammal.

2. The compound for use in the diagnosis method according to claim 1, wherein said compound comprises a peptide.

3. The compound for use in the diagnosis method according to claim 2, wherein said peptide is selected from the group consisting of human FcαR1 (SEQ ID NO:2), human Fcα/µ receptor (SEQ ID NO:6), polymeric Ig receptor (SEQ ID NO:7), Sir22 (SEQ ID NO:3), streptococcal IgA-binding peptide (Sap; SEQ ID NO:4), a modified Z-domain protein (SEQ ID NO: 12), and YDWIPSSAW (SEQ ID NO:9), or an IgA- or IgM-binding fragment thereof.

4. The compound for use in the diagnosis method according to claim 2 or 3, wherein said peptide comprises a modification selected from the group consisting of an N-terminal cap, a C-terminal cap, a D-amino acid, an amino acid surrogate, a peptidomimetic sequence, and a spacer.

5. The compound for use in the diagnosis method according to claim 1, wherein said compound comprises an antibody, or an IgA- or IgM-binding fragment thereof, said antibody being preferably anti-human IgA, or an IgA-binding fragment thereof, or anti-human IgM, or an IgM-binding fragment thereof.

6. The compound for use in the diagnosis method according to any one of claims 1 to 5, wherein said radiolabel on the second member of the binding pair is selected from the group consisting of ^{99m}Tc, ¹¹¹In, ⁶⁶Ga, ⁶⁷Ga, ⁶⁸Ga, ⁸⁶Y, ⁹⁰Y, ²⁰¹TI, ⁵⁵Co, ⁶⁰Cu, ⁶¹Cu, ⁶²Cu, ⁶⁴Cu, ⁶⁷Cu, ⁸²Rb, ^{185/187}Re, and ^{186/188}Re.

7. The compound for use in the diagnosis method according to any one of claims 1 to 6, wherein said detection is carried out by an imaging technique, preferably one of SPECT, PET, planar scan, and MRI.

8. The compound for use in the diagnosis method according to any one of claims 1 to 7, wherein the first member of the binding pair is selected from either streptavidin or biotin.

9. The compound for use in the diagnosis method according to any one of claims 1 to 8, wherein said administration further comprises administration of galactose-Ficoll.

10. The compound for use in the diagnosis method according to any one of claims 1 to 9, wherein said second member of the binding pair is linked to both a galactose, and a radioactively labeled compound, said radioactively labelled compound being preferably labeled with an isotope selected from the group consisting of ^{99m}Tc, ¹¹¹In, ⁶⁶Ga, ⁶⁷Ga, ⁶⁸Ga, ⁸⁶Y, ⁹⁰Y, ²⁰¹TI, ⁵⁵Co, ⁶⁰Cu, ⁶¹Cu, ⁶²Cu, ⁶⁴Cu, ⁶⁷Cu, ⁸²Rb, ^{185/187}Re, and ^{186/188}Re.

11. The compound for use in the diagnosis method according to claim 10, wherein said radiolabeled compound is human serum albumin.

12. The compound for use in the diagnosis method according to any one of claims 1 to 11, wherein said second member of a binding pair is selected from either biotin or streptavidin such that it is not the same as the first member of the binding pair.

13. A kit comprising:
(a) an IgA- or IgM-binding compound wherein the compound is linked to a first member of a binding pair and to galactose;
(b) a radiolabelled substance linked to a second member of the binding pair and to galactose; and
(c) instructions for use for detecting an IgA or IgM kidney disease in a mammal.

14. The kit of claim 13, wherein said second member of the binding pair comprises a radioactively labeled peptide, said radiolabel being preferably selected from the group consisting of ^{99m}Tc, ¹¹¹In, ⁶⁶Ga, ⁶⁷Ga, ⁶⁸Ga, ⁸⁶Y, ⁹⁰Y, ²⁰¹TI, ⁵⁵Co, ⁶⁰Cu, ⁶¹Cu, ⁶²Cu, ⁶⁴Cu, ⁶⁷Cu, ⁸²Rb, ^{185/187}Re, and ^{186/188}Re.

15. The kit of claim 13 or 14, wherein said first member of the binding pair is streptavidin, and said second member of the binding pair is biotin.

16. The kit of claim 13 or 14, wherein said radioactively labeled peptide is human serum albumin.

17. The kit of any one of claims 13 to 16, additionally comprising galactose-Ficoll.

## Patentansprüche

1. Verbindung, die IgA oder IgM spezifisch bindet, für die in-vivo-Diagnose einer IgA- oder IgM-Nierenkrankheit in einem Säuger, vorzugsweise einem Menschen, wobei die Verbindung an ein erstes Element eines Bindungspaares und an Galactose gebunden ist, wobei die Verbindung in der Niere des Säugers durch eine radioaktiv markierte Substanz nachgewiesen wird, die an ein zweites Element des Bindungspaares und an Galactose gebunden ist, wobei der Nachweis durchgeführt wird, nachdem ungebundene Verbindung aus dem Blutkreislauf entfernt worden ist; und wobei eine Erhöhung der Menge der Verbindung in der Niere des Säugers, bezogen auf die Menge in der Niere eines Säugers ohne die Nierenkrankheit, für die IgA- oder IgM-Nierenkrankheit in dem Säuger diagnostisch ist.

2. Verbindung für die Verwendung in dem Diagnoseverfahren nach Anspruch 1, wobei die Verbindung ein Peptid enthält.

3. Verbindung für die Verwendung in dem Diagnoseverfahren nach Anspruch 2, wobei das Peptid aus der Gruppe ausgewählt ist, die aus menschlichem FcαR1 (SEQ ID NO:2), menschlichem Fcα/µ Rezeptor (SEQ ID NO:6), polymerem Ig Rezeptor (SEQ ID NO:7), Sir22 (SEQ ID NO:3), Streptokokken IgA-Bindungspeptid (Sap; SEQ ID NO:4), einem modifizierten Z-Domainenprotein (SEQ ID NO:12) und YDWIPSSAW (SEQ ID NO:9) besteht, oder ein IgA- oder IgM-bindendes Fragment davon ist.

4. Verbindung für die Verwendung in dem Diagnoseverfahren nach Anspruch 2 oder 3, wobei das Peptid eine Modifikation umfasst, die aus der Gruppe ausgewählt ist, die aus einer N-terminalen Kappe, einer C-terminalen Kappe, einer D-Aminosäure, einem Aminosäuresurrogat, einer peptidomimetischen Sequenz oder einem Abstandshalter besteht.

5. Verbindung für die Verwendung in dem Diagnoseverfahren nach Anspruch 1, wobei die Verbindung einen Antikörper oder einen IgA- oder IgM-bindendes Fragment davon enthält, wobei der Antikörper vorzugsweise Anti-Mensch-IgA oder ein IgA-bindendes Fragment davon oder Anti-Mensch-IgM oder ein IgMbindendes Fragment davon ist.

6. Verbindung für die Verwendung in dem Diagnoseverfahren nach einem der Ansprüche 1 bis 5, wobei die radioaktive Markierung in dem zweiten Element des Bindungspaares aus der Gruppe ausgewählt ist, die aus ^{99m}Tc, ¹¹¹In, ⁶⁶Ga, ⁶⁷Ga, ⁶⁸Ga, ⁸⁶Y, ⁹⁰Y, ²⁰¹TI, ⁵⁵Co, ⁶⁰Cu, ⁶¹Cu, ⁶²Cu, ⁶⁴Cu, ⁶⁷Cu, ⁸²Rb, ^{185/187}Re und ^{186/188}Re besteht.

7. Verbindung für die Verwendung in dem Diagnoseverfahren nach einem der Ansprüche 1 bis 6, wobei der Nachweis durch ein Bildgebungsverfahren, beispielsweise SPECT, PET, planares Abtasten und MRI, durchgeführt wird.

8. Verbindung für die Verwendung in dem Diagnoseverfahren nach einem der Ansprüche 1 bis 7, wobei das erste Element des Bindungspaares entweder unter Streptavidin oder Biotin ausgewählt ist.

9. Verbindung für die Verwendung in dem Diagnoseverfahren nach einem der Ansprüche 1 bis 8, wobei die Verabreichung außerdem die Verabreichung von Galactose-Ficoll umfasst.

10. Verbindung für die Verwendung in dem Diagnoseverfahren nach einem der Ansprüche 1 bis 9, wobei das zweite Element des Bindungspaares sowohl an Galactose als auch an eine radioaktiv markierte Verbindung gebunden ist, wobei die radioaktiv markierte Verbindung vorzugsweise mit einem Isotop markiert ist, das aus der Gruppe ausgewählt ist, die aus ^{99m}Tc, ¹¹¹In, ⁶⁶Ga, ⁶⁷Ga, ⁶⁸Ga, ⁸⁶Y, ⁹⁰Y, ²⁰¹TI, ⁵⁵Co, ⁶⁰Cu, ⁶¹Cu, ⁶²Cu, ⁶⁴Cu, ⁶⁷Cu, ⁸²Rb, ^{185/187}Re und ^{186/188}Re besteht.

11. Verbindung für die Verwendung in dem Diagnoseverfahren nach Anspruch 10, wobei die radioaktiv markierte Verbindung menschliches Serumalbumin ist.

12. Verbindung für die Verwendung in dem Diagnoseverfahren nach einem der Ansprüche 1 bis 11, wobei das zweite Element des Bindungspaares entweder aus Biotin oder Streptavidin ausgewählt ist, so dass es nicht dasselbe ist wie das erste Element des Bindungspaares.

13. Kit, welcher umfasst:
(a) eine IgA- oder IgM-bindende Verbindung, wobei die Verbindung an ein erstes Element eines Bindungspaares und an Galactose gebunden ist;
(b) eine radioaktiv markierte Substanz, die an ein zweites Element des Bindungspaares und an Galactose gebunden ist, und
(c) Anweisungen für die Verwendung zum Nachweisen einer IgA- oder IgM-Nierenkrankheit in einem Säuger.

14. Kit nach Anspruch 13, wobei das zweite Element des Bindungspaares ein radioaktiv markiertes Peptid umfasst, wobei die radioaktive Markierung vorzugsweise aus der Gruppe ausgewählt ist die aus ^{99m}Tc, ¹¹¹In, ⁶⁶Ga, ⁶⁷Ga, ⁶⁸Ga, ⁸⁶Y, ⁹⁰Y, ²⁰¹TI, ⁵⁵Co, ⁶⁰Cu, ⁶¹Cu, ⁶²Cu, ⁶⁴Cu, ⁶⁷Cu, ⁸²Rb, ^{185/187}Re und ^{186/188}Re besteht.

15. Kit nach Anspruch 13 oder 14, wobei das erste Element des Bindungspaares Streptavidin ist und das zweite Element des Bindungspaares Biotin ist.

16. Kit nach Anspruch 13 oder 14, wobei das radioaktiv markierte Peptid menschliches Serumalbumin ist.

17. Kit nach einem der Ansprüche 13 bis 16, der außerdem Galactose-Ficoll enthält.

## Revendications

1. Composé qui se lie spécifiquement à une IgA ou une IgM destiné à être utilisé dans le diagnostic *in vivo* d'une maladie rénale à IgA ou IgM chez un mammifère, de préférence un humain, le composé étant lié à un premier membre d'une paire de liaison et au galactose, où le composé est détecté dans le rein dudit mammifère par une substance radiomarquée liée à un second membre de la paire de liaison et au galactose, où la détection a lieu suite à la clairance du composé non lié de la circulation ; et une augmentation de la quantité dudit composé dans le rein dudit mammifère par rapport à la quantité dans le rein d'un mammifère ne présentant pas ladite maladie rénale permet d'établir le diagnostic de ladite maladie rénale à IgA ou IgM chez ledit mammifère.

2. Composé destiné à être utilisé dans le procédé de diagnostic selon la revendication 1, où ledit composé comprend un peptide.

3. Composé destiné à être utilisé dans le procédé de diagnostic selon la revendication 2, où ledit peptide est sélectionné dans le groupe consistant en le FcαR1 humain (SEQ ID NO: 2), le récepteur Fcα/µ humain (SEQ ID NO: 6), un récepteur d'Ig polymérique (SEQ ID NO: 7), Sir22 (SEQ ID NO: 3), un peptide streptococcique se liant à une IgA (Sap ; SEQ ID NO: 4), une protéine à domaine Z modifiée (SEQ ID NO: 12), et YDWIPSSAW (SEQ ID NO: 9), ou un fragment de ceux-ci se liant à une IgA ou une IgM.

4. Composé destiné à être utilisé dans le procédé de diagnostic selon la revendication 2 ou 3, où ledit peptide comprend une modification sélectionnée dans le groupe consistant en une coiffe N-terminale, une coiffe C-terminale, un acide D-aminé, un substitut d'acide aminé, une séquence peptidomimétique, et un espaceur.

5. Composé destiné à être utilisé dans le procédé de diagnostic selon la revendication 1, où ledit composé comprend un anticorps, ou un fragment de celui-ci se liant à une IgA ou une IgM, ledit anticorps étant de préférence un anticorps anti-IgA humaine, ou un fragment de celui-ci se liant à une IgA, ou un anticorps anti-IgM humaine, ou un fragment de celui-ci se liant à une IgM.

6. Composé destiné à être utilisé dans le procédé de diagnostic selon l'une quelconque des revendications 1 à 5, dans lequel ledit radiomarqueur sur le second membre de la paire de liaison est sélectionné dans le groupe consistant en ^{99m}Tc, ¹¹¹In, ⁶⁶Ga, ⁶⁷Ga, ⁶⁸Ga, ⁸⁶Y, ⁹⁰Y, ²⁰¹TI, ⁵⁵Co, ⁶⁰Cu, ⁶¹Cu, ⁶²Cu, ^{64C}u, ⁶⁷Cu, ⁸²Rb, ^{185/187}Re, et ^{186/188}Re.

7. Composé destiné à être utilisé dans le procédé de diagnostic selon l'une quelconque des revendications 1 à 6, où ladite détection est réalisée par une technique d'imagerie, de préférence sélectionnée parmi TEMP, TEP, scintigraphie planaire, et IRM.

8. Composé destiné à être utilisé dans le procédé de diagnostic selon l'une quelconque des revendications 1 à 7, où ledit premier membre de la paire de liaison est sélectionné parmi la streptavidine ou la biotine.

9. Composé destiné à être utilisé dans le procédé de diagnostic selon l'une quelconque des revendications 1 à 8, où ladite administration comprend en outre l'administration de galactose-Ficoll.

10. Composé destiné à être utilisé dans le procédé de diagnostic selon l'une quelconque des revendications 1 à 9, où ledit second membre de la paire de liaison est lié à la fois au galactose et à un composé marqué radioactivement, ledit composé marqué par la radioactivité étant de préférence marqué avec un isotope sélectionné dans le groupe consistant en ^{99m}Tc, ¹¹¹In, ⁶⁶Ga, ⁶⁷Ga, ⁶⁸Ga, ⁸⁶Y, ⁹⁰Y, ²⁰¹TI' ⁵⁵Co, ⁶¹Cu, ⁶¹Cu, ⁶²Cu, ⁶⁴Cu, ⁶⁷Cu, ⁸²Rb, ^{185/187}Re, et ^{186/188}Re.

11. Composé destiné à être utilisé dans le procédé de diagnostic selon la revendication 10, où ledit composé radiomarqué est la sérumalbumine humaine.

12. Composé destiné à être utilisé dans le procédé de diagnostic selon l'une quelconque des revendications 1 à 11, dans lequel ledit second membre de la paire de liaison est sélectionné parmi la biotine ou la streptavidine de sorte qu'il ne soit pas le même que le premier membre de la paire de liaison.

13. Kit comprenant :
(a) un composé se liant à une IgA ou une IgM, où le composé est lié à un premier membre d'une paire de liaison et au galactose ;
(b) une substance radiomarquée liée à un second membre de la paire de liaison et au galactose ; et
(c) un mode d'emploi pour détecter une maladie rénale à IgA ou IgM chez un mammifère.

14. Kit selon la revendication 13, dans lequel ledit second membre de la paire de liaison comprend un peptide marqué par la radioactivité, ledit radiomarqueur étant de préférence sélectionné dans le groupe consistant en ^{99m} Tc, ¹¹¹In, ⁶⁶Ga, ⁶⁷Ga, ⁶⁸Ga, ⁸⁶Y, ⁹⁰Y, ²⁰¹TI' ⁵⁵Co, ⁶⁰Cu, ⁶¹Cu, ⁶²Cu, ⁶⁴Cu, ⁶⁷Cu, ⁸²Rb, ^{185/187}Re, et ^{186/188}Re.

15. Kit selon la revendication 13 ou 14, dans lequel ledit premier membre de la paire de liaison est la streptavidine, et ledit second membre de la paire de liaison est la biotine.

16. Kit selon la revendication 13 ou 14, dans lequel ledit peptide marqué par la radioactivité est la sérumalbumine humaine.

17. Kit selon l'une quelconque des revendications 13 à 16, comprenant en outre du galactose-Ficoll.


### SEQUENCE LISTING

<110> RQ Bioscience, Inc. New England Medical Center Hospitals, Inc.
<120> Methods and Compositions For Diagnosis of IgA- and IgM-Mediated Kidney Diseases
<130> 50400/002WO2
<150> US 60/705,282
   <151> 2005-08-03
<160> 17
<170> PatentIn version 3.3
<210> 1
   <211> 353
   <212> PRT
   <213> Homo sapiens
<400> 1
   Ala Ser Pro Thr Ser Pro Lys Val Phe Pro Leu Ser Leu Cys Ser Thr
   1               5                   10                  15
   Gln Pro Asp Gly Asn Val Val Ile Ala Cys Leu Val Gln Gly Phe Phe
               20                  25                  30
   Pro Gln Glu Pro Leu Ser Val Thr Trp Ser Glu Ser Gly Gln Gly Val
           35                  40                  45
   Thr Ala Arg Asn Phe Pro Pro Ser Gln Asp Ala Ser Gly Asp Leu Tyr
       50                  55                  60
   Thr Thr Ser Ser Gln Leu Thr Leu Pro Ala Thr Gln Cys Leu Ala Gly
   65                  70                  75                  80
   Lys Ser Val Thr Cys His Val Lys His Tyr Thr Asn Pro Ser Gln Asp
                   85                  90                  95
   Val Thr Val Pro Cys Pro Val Pro Ser Thr Pro Pro Thr Pro Ser Pro
               100                 105                 110
   Ser Thr Pro Pro Thr Pro Ser Pro Ser Cys Cys His Pro Arg Leu Ser
           115                 120                 125
   Leu His Arg Pro Ala Leu Glu Asp Leu Leu Leu Gly Ser Glu Ala Asn
       130                 135                 140
   Leu Thr Cys Thr Leu Thr Gly Leu Arg Asp Ala Ser Gly Val Thr Phe
   145                 150                 155                 160
   Thr Trp Thr Pro Ser Ser Gly Lys Ser Ala Val Gln Gly Pro Pro Glu
                   165                 170                 175
   Arg Asp Leu Cys Gly Cys Tyr Ser Val Ser Ser Val Leu Pro Gly Cys
               180                 185                 190
   Ala Glu Pro Trp Asn His Gly Lys Thr Phe Thr Cys Thr Ala Ala Tyr
           195                 200                 205
   Pro Glu Ser Lys Thr Pro Leu Thr Ala Thr Leu Ser Lys Ser Gly Asn
       210                 215                 220
   Thr Phe Arg Pro Glu Val His Leu Leu Pro Pro Pro Ser Glu Glu Leu
   225                 230                 235                 240
   Ala Leu Asn Glu Leu Val Thr Leu Thr Cys Leu Ala Arg Gly Phe Ser
                   245                 250                 255
   Pro Lys Asp Val Leu Val Arg Trp Leu Gln Gly Ser Gln Glu Leu Pro
               260                 265                 270
   Arg Glu Lys Tyr Leu Thr Trp Ala Ser Arg Gln Glu Pro Ser Gln Gly
           275                 280                 285
   Thr Thr Thr Phe Ala Val Thr Ser Ile Leu Arg Val Ala Ala Glu Asp
       290                 295                 300
   Trp Lys Lys Gly Asp Thr Phe Ser Cys Met Val Gly His Glu Ala Leu
   305                 310                 315                 320
   Pro Leu Ala Phe Thr Gln Lys Thr Ile Asp Arg Leu Ala Gly Lys Pro
                   325                 330                 335
   Thr His Val Asn Val Ser Val Val Met Ala Glu Val Asp Gly Thr Cys
               340                 345                 350
   Tyr
<210> 2
   <211> 192
   <212> PRT
   <213> Homo sapiens
<220>
   <221> misc_feature
   <222> (140)..(140)
   <223> Xaa can be any naturally occurring amino acid
<400> 2
   Asp Phe Pro Met Pro Phe Ile Ser Ala Lys Ser Ser Pro Val Ile Pro
   1               5                   10                  15
   Leu Asp Gly Ser Val Lys Ile Gln Cys Gln Ala Ile Arg Glu Ala Tyr
               20                  25                  30
   Leu Thr Gln Leu Met Ile Ile Lys Asn Ser Thr Tyr Arg Glu Ile Gly
           35                  40                  45
   Arg Arg Leu Lys Phe Trp Asn Glu Thr Asp Pro Glu Phe Val Ile Asp
       50                  55                  60
   His Met Asp Ala Asn Lys Ala Gly Arg Tyr Gln Cys Gln Tyr Arg Ile
   65                  70                  75                  80
   Gly His Tyr Arg Phe Arg Tyr Ser Asp Thr Leu Glu Leu Val Val Thr
                   85                  90                  95
   Gly Leu Tyr Gly Lys Pro Phe Leu Ser Ala Asp Arg Gly Leu Val Leu
               100                 105                 110
   Met Pro Gly Glu Asn Ile Ser Leu Thr Cys Ser Ser Ala His Ile Pro
           115                 120                 125
   Phe Asp Arg Phe Ser Leu Ala Lys Glu Gly Glu Xaa Ser Leu Pro Gln
       130                 135                 140
   His Gln Ser Gly Glu His Pro Ala Asn Phe Ser Leu Gly Pro Val Asp
   145                 150                 155                 160
   Leu Asn Val Ser Gly Ile Tyr Arg Cys Tyr Gly Trp Tyr Asn Arg Ser
                   165                 170                 175
   Pro Tyr Leu Trp Ser Phe Pro Ser Asn Ala Leu Glu Leu Val Val Thr
               180                 185                 190
<210> 3
   <211> 365
   <212> PRT
   <213> Streptococcus pyogenes
<400> 3
   Met Ala Arg Lys Asp Thr Asn Lys Gln Tyr Ser Leu Arg Lys Leu Lys
   1               5                   10                  15
   Thr Gly Thr Ala Ser Val Ala Val Ala Val Ala Val Leu Gly Ala Gly
               20                  25                  30
   Phe Ala Asn Gln Thr Thr Val Lys Ala Glu Ser Ser Asn Asn Ala Glu
           35                  40                  45
   Ser Ser Asn Ile Ser Gln Glu Ser Lys Leu Ile Asn Thr Leu Thr Asp
       50                  55                  60
   Glu Asn Glu Lys Leu Arg Glu Glu Leu Gln Gln Tyr Tyr Ala Leu Ser
   65                  70                  75                  80
   Asp Ala Lys Glu Glu Glu Pro Arg Tyr Lys Ala Leu Arg Gly Glu Asn
                   85                  90                  95
   Gln Asp Leu Arg Glu Lys Glu Arg Lys Tyr Gln Asp Lys Ile Lys Lys
               100                 105                 110
   Leu Glu Glu Lys Glu Lys Asn Leu Glu Lys Lys Ser Glu Asp Val Glu
           115                 120                 125
   Arg His Tyr Leu Lys Lys Leu Asp Gln Glu His Lys Glu Gln Gln Glu
       130                 135                 140
   Arg Gln Lys Asn Leu Glu Glu Leu Glu Arg Gln Ser Gln Arg Glu Ile
   145                 150                 155                 160
   Asp Lys Arg Tyr Gln Glu Gln Leu Gln Lys Gln Gln Gln Leu Glu Thr
                   165                 170                 175
   Glu Lys Gln Ile Ser Glu Ala Ser Arg Lys Ser Leu Ser Arg Asp Leu
               180                 185                 190
   Glu Ala Ser Arg Ala Ala Lys Lys Lys Val Glu Ala Asp Leu Ala Ala
           195                 200                 205
   Leu Asn Ala Glu His Gln Lys Leu Lys Glu Glu Lys Gln Ile Ser Asp
       210                 215                 220
   Ala Ser Arg Gln Gly Leu Ser Arg Asp Leu Glu Ala Ser Arg Glu Ala
   225                 230                 235                 240
   Lys Lys Lys Val Glu Ala Asp Leu Ala Glu Ala Asn Ser Lys Leu Gln
                   245                 250                 255
   Ala Leu Glu Lys Leu Asn Lys Glu Leu Glu Glu Gly Lys Lys Leu Ser
               260                 265                 270
   Glu Lys Glu Lys Ala Glu Leu Gln Ala Arg Leu Glu Ala Glu Ala Lys
           275                 280                 285
   Ala Leu Lys Glu Gln Leu Ala Lys Gln Ala Glu Glu Leu Ala Lys Leu
       290                 295                 300
   Lys Gly Asn Gln Thr Pro Asn Ala Lys Val Ala Pro Gln Ala Asn Arg
   305                 310                 315                 320
   Ser Arg Ser Ala Met Thr Gln Gln Lys Arg Thr Leu Pro Ser Thr Gly
                   325                 330                 335
   Glu Ala Ala Asn Pro Phe Phe Thr Ala Ala Ala Ala Thr Val Met Val
               340                 345                 350
   Ser Ala Gly Met Leu Ala Leu Lys Arg Lys Glu Glu Asn
           355                 360                 365
<210> 4
   <211> 50
   <212> PRT
   <213> Artificial sequence
<220>
   <223> Sap
<400> 4
   Tyr Tyr Ala Leu Ser Asp Ala Lys Glu Glu Glu Pro Arg Tyr Lys Ala
   1 5 10 15
   Leu Arg Gly Glu Asn Gln Asp Leu Arg Glu Lys Glu Arg Lys Tyr Gln
   20 25 30
   Asp Lys Ile Lys Lys Leu Glu Glu Lys Glu Lys Asn Leu Glu Lys Lys
   35 40 45
   Ser Cys
   50
<210> 5
   <211> 287
   <212> PRT
   <213> Homo sapiens
<400> 5
   Met Asp Pro Lys Gln Thr Thr Leu Leu Cys Leu Val Leu Cys Leu Gly
   1 5 10 15
   Gln Arg Ile Gln Ala Gln Glu Gly Asp Phe Pro Met Pro Phe Ile Ser
   20 25 30
   Ala Lys Ser Ser Pro Val Ile Pro Leu Asp Gly Ser Val Lys Ile Gln
   35 40 45
   Cys Gln Ala Ile Arg Glu Ala Tyr Leu Thr Gln Leu Met Ile Ile Lys
   50 55 60
   Asn Ser Thr Tyr Arg Glu Ile Gly Arg Arg Leu Lys Phe Trp Asn Glu
   65 70 75 80
   Thr Asp Pro Glu Phe Val Ile Asp His Met Asp Ala Asn Lys Ala Gly
   85 90 95
   Arg Tyr Gln Cys Gln Tyr Arg Ile Gly His Tyr Arg Phe Arg Tyr Ser
   100 105 110
   Asp Thr Leu Glu Leu Val Val Thr Gly Leu Tyr Gly Lys Pro Phe Leu
   115 120 125
   Ser Ala Asp Arg Gly Leu Val Leu Met Pro Gly Glu Asn Ile Ser Leu
   130 135 140
   Thr Cys Ser Ser Ala His Ile Pro Phe Asp Arg Phe Ser Leu Ala Lys
   145 150 155 160
   Glu Gly Glu Leu Ser Leu Pro Gln His Gln Ser Gly Glu His Pro Ala
   165 170 175
   Asn Phe Ser Leu Gly Pro Val Asp Leu Asn Val Ser Gly Ile Tyr Arg
   180 185 190
   Cys Tyr Gly Trp Tyr Asn Arg Ser Pro Tyr Leu Trp Ser Phe Pro Ser
   195 200 205
   Asn Ala Leu Glu Leu Val Val Thr Asp Ser Ile His Gln Asp Tyr Thr
   210 215 220
   Thr Gln Asn Leu Ile Arg Met Ala Val Ala Gly Leu Val Leu Val Ala
   225 230 235 240
   Leu Leu Ala Ile Leu Val Glu Asn Trp His Ser His Thr Ala Leu Asn
   245 250 255
   Lys Glu Ala Ser Ala Asp Val Ala Glu Pro Ser Trp Ser Gln Gln Met
   260 265 270
   Cys Gln Pro Gly Leu Thr Phe Ala Arg Thr Pro Ser Val Cys Lys
   275 280 285
<210> 6
   <211> 534
   <212> PRT
   <213> Homo sapiens
<400> 6
   Met Asp Gly Glu Ala Thr Val Lys Pro Gly Glu Gln Val Pro Leu Trp
   1 5 10 15
   Thr His Gly Trp Pro Pro Asp Asp Pro Ser Pro Ser Phe Ala Ala Gly
               20                  25                  30
   Ser Ser Phe Ala Leu Pro Gln Lys Arg Pro His Pro Arg Trp Leu Trp
           35                  40                  45
   Glu Gly Ser Leu Pro Ser Arg Thr His Leu Arg Ala Met Gly Thr Leu
       50                  55                  60
   Arg Pro Ser Ser Pro Leu Cys Trp Arg Glu Glu Ser Ser Phe Ala Ala
   65                  70                  75                  80
   Pro Asn Ser Leu Lys Gly Ser Arg Leu Val Ser Gly Glu Pro Gly Gly
                   85                  90                  95
   Ala Val Thr Ile Gln Cys His Tyr Ala Pro Ser Ser Val Asn Arg His
               100                 105                 110
   Gln Arg Lys Tyr Trp Cys Arg Leu Gly Pro Pro Arg Trp Ile Cys Gln
           115                 120                 125
   Thr Ile Val Ser Thr Asn Gln Tyr Thr His His Arg Tyr Arg Asp Arg
       130                 135                 140
   Val Ala Leu Thr Asp Phe Pro Gln Arg Gly Leu Phe Val Val Arg Leu
   145                 150                 155                 160
   Ser Gln Leu Ser Pro Asp Asp Ile Gly Cys Tyr Leu Cys Gly Ile Gly
                   165                 170                 175
   Ser Glu Asn Asn Met Leu Phe Leu Ser Met Asn Leu Thr Ile Ser Ala
               180                 185                 190
   Gly Pro Ala Ser Thr Leu Pro Thr Ala Thr Pro Ala Ala Gly Glu Leu
           195                 200                 205
   Thr Met Arg Ser Tyr Gly Thr Ala Ser Pro Val Ala Asn Arg Trp Thr
       210                 215                 220
   Pro Gly Thr Thr Gln Thr Leu Gly Gln Gly Thr Ala Trp Asp Thr Val
   225                 230                 235                 240
   Ala Ser Thr Pro Gly Thr Ser Lys Thr Thr Ala Ser Ala Glu Gly Arg
                   245                 250                 255
   Arg Thr Pro Gly Ala Thr Arg Pro Ala Ala Pro Gly Thr Gly Ser Trp
               260                 265                 270
   Ala Glu Gly Ser Val Lys Ala Pro Ala Pro Ile Pro Glu Ser Pro Pro
           275                 280                 285
   Ser Lys Ser Arg Ser Met Ser Asn Thr Thr Glu Gly Val Trp Glu Gly
       290                 295                 300
   Thr Arg Ser Ser Val Thr Asn Arg Ala Arg Ala Ser Lys Asp Arg Arg
   305                 310                 315                 320
   Glu Met Thr Thr Thr Lys Ala Asp Arg Pro Arg Glu Asp Ile Glu Gly
                   325                 330                 335
   Val Arg Ile Ala Leu Asp Ala Ala Lys Lys Val Leu Gly Thr Ile Gly
               340                 345                 350
   Pro Pro Ala Leu Val Ser Glu Thr Leu Ala Trp Glu Ile Leu Pro Gln
           355                 360                 365
   Ala Thr Pro Val Ser Lys Gln Gln Ser Gln Gly Ser Ile Gly Glu Thr
       370                 375                 380
   Thr Pro Ala Ala Gly Met Trp Thr Leu Gly Thr Pro Ala Ala Asp Val
   385                 390                 395                 400
   Trp Ile Thr Ser Met Glu Ala Ala Ser Gly Glu Gly Ser Ala Ala Gly
                   405                 410                 415
   Asp Leu Asp Ala Ala Thr Gly Asp Arg Gly Pro Gln Ala Thr Leu Ser
               420                 425                 430
   Gln Thr Pro Ala Val Gly Pro Trp Gly Pro Pro Gly Lys Glu Ser Ser
           435                 440                 445
   Val Lys Arg Ala Thr Cys Pro Gly Leu Ser Thr Leu Lys Glu Ile Lys
       450                 455                 460
   Val Ser Ala Val Leu Thr Gln Asn Pro His Ser Leu Leu Cys Val Ser
   465 470 475 480
   Ala Ala Gln Glu Ala Glu Arg Val Thr Leu Ile Gln Met Thr His Phe
   485 490 495
   Leu Glu Val Asn Pro Gln Ala Asp Gln Leu Pro His Val Glu Arg Lys
   500 505 510
   Met Leu Gln Asp Asp Ser Leu Pro Ala Gly Ala Ser Leu Thr Ala Pro
   515 520 525
   Glu Arg Asn Pro Gly Pro
   530
<210> 7
   <211> 764
   <212> PRT
   <213> Homo sapiens
<400> 7
   Met Leu Leu Phe Val Leu Thr Cys Leu Leu Ala Val Phe Pro Ala Ile
   1 5 10 15
   Ser Thr Lys Ser Pro Ile Phe Gly Pro Glu Glu Val Asn Ser Val Glu
   20 25 30
   Gly Asn Ser Val Ser Ile Thr Cys Tyr Tyr Pro Pro Thr Ser Val Asn
   35 40 45
   Arg His Thr Arg Lys Tyr Trp Cys Arg Gln Gly Ala Arg Gly Gly Cys
   50 55 60
   Ile Thr Leu Ile Ser Ser Glu Gly Tyr Val Ser Ser Lys Tyr Ala Gly
   65 70 75 80
   Arg Ala Asn Leu Thr Asn Phe Pro Glu Asn Gly Thr Phe Val Val Asn
   85 90 95
   Ile Ala Gln Leu Ser Gln Asp Asp Ser Gly Arg Tyr Lys Cys Gly Leu
   100 105 110
   Gly Ile Asn Ser Arg Gly Leu Ser Phe Asp Val Ser Leu Glu Val Ser
   115 120 125
   Gln Gly Pro Gly Leu Leu Asn Asp Thr Lys Val Tyr Thr Val Asp Leu
   130 135 140
   Gly Arg Thr Val Thr Ile Asn Cys Pro Phe Lys Thr Glu Asn Ala Gln
   145 150 155 160
   Lys Arg Lys Ser Leu Tyr Lys Gln Ile Gly Leu Tyr Pro Val Leu Val
   165 170 175
   Ile Asp Ser Ser Gly Tyr Val Asn Pro Asn Tyr Thr Gly Arg Ile Arg
   180 185 190
   Leu Asp Ile Gln Gly Thr Gly Gln Leu Leu Phe Ser Val Val Ile Asn
   195 200 205
   Gln Leu Arg Leu Ser Asp Ala Gly Gln Tyr Leu Cys Gln Ala Gly Asp
   210 215 220
   Asp Ser Asn Ser Asn Lys Lys Asn Ala Asp Leu Gln Val Leu Lys Pro
   225 230 235 240
   Glu Pro Glu Leu Val Tyr Glu Asp Leu Arg Gly Ser Val Thr Phe His
   245 250 255
   Cys Ala Leu Gly Pro Glu Val Ala Asn Val Ala Lys Phe Leu Cys Arg
   260 265 270
   Gln Ser Ser Gly Glu Asn Cys Asp Val Val Val Asn Thr Leu Gly Lys
   275 280 285
   Arg Ala Pro Ala Phe Glu Gly Arg Ile Leu Leu Asn Pro Gln Asp Lys
   290 295 300
   Asp Gly Ser Phe Ser Val Val Ile Thr Gly Leu Arg Lys Glu Asp Ala
   305 310 315 320
   Gly Arg Tyr Leu Cys Gly Ala His Ser Asp Gly Gln Leu Gln Glu Gly
   325 330 335
   Ser Pro Ile Gln Ala Trp Gln Leu Phe Val Asn Glu Glu Ser Thr Ile
   340 345 350
   Pro Arg Ser Pro Thr Val Val Lys Gly Val Ala Gly Gly Ser Val Ala
   355 360 365
   Val Leu Cys Pro Tyr Asn Arg Lys Glu Ser Lys Ser Ile Lys Tyr Trp
   370 375 380
   Cys Leu Trp Glu Gly Ala Gln Asn Gly Arg Cys Pro Leu Leu Val Asp
   385 390 395 400
   Ser Glu Gly Trp Val Lys Ala Gln Tyr Glu Gly Arg Leu Ser Leu Leu
   405 410 415
   Glu Glu Pro Gly Asn Gly Thr Phe Thr Val Ile Leu Asn Gln Leu Thr
   420 425 430
   Ser Arg Asp Ala Gly Phe Tyr Trp Cys Leu Thr Asn Gly Asp Thr Leu
   435 440 445
   Trp Arg Thr Thr Val Glu Ile Lys Ile Ile Glu Gly Glu Pro Asn Leu
   450 455 460
   Lys Val Pro Gly Asn Val Thr Ala Val Leu Gly Glu Thr Leu Lys Val
   465 470 475 480
   Pro Cys His Phe Pro Cys Lys Phe Ser Ser Tyr Glu Lys Tyr Trp Cys
   485 490 495
   Lys Trp Asn Asn Thr Gly Cys Gln Ala Leu Pro Ser Gln Asp Glu Gly
   500 505 510
   Pro Ser Lys Ala Phe Val Asn Cys Asp Glu Asn Ser Arg Leu Val Ser
   515 520 525
   Leu Thr Leu Asn Leu Val Thr Arg Ala Asp Glu Gly Trp Tyr Trp Cys
   530 535 540
   Gly Val Lys Gln Gly His Phe Tyr Gly Glu Thr Ala Ala Val Tyr Val
   545 550 555 560
   Ala Val Glu Glu Arg Lys Ala Ala Gly Ser Arg Asp Val Ser Leu Ala
   565 570 575
   Lys Ala Asp Ala Ala Pro Asp Glu Lys Val Leu Asp Ser Gly Phe Arg
   580 585 590
   Glu Ile Glu Asn Lys Ala Ile Gln Asp Pro Arg Leu Phe Ala Glu Glu
   595 600 605
   Lys Ala Val Ala Asp Thr Arg Asp Gln Ala Asp Gly Ser Arg Ala Ser
   610 615 620
   Val Asp Ser Gly Ser Ser Glu Glu Gln Gly Gly Ser Ser Arg Ala Leu
   625 630 635 640
   Val Ser Thr Leu Val Pro Leu Gly Leu Val Leu Ala Val Gly Ala Val
   645 650 655
   Ala Val Gly Val Ala Arg Ala Arg His Arg Lys Asn Val Asp Arg Val
   660 665 670
   Ser Ile Arg Ser Tyr Arg Thr Asp Ile Ser Met Ser Asp Phe Glu Asn
   675 680 685
   Ser Arg Glu Phe Gly Ala Asn Asp Asn Met Gly Ala Ser Ser Ile Thr
   690 695 700
   Gln Glu Thr Ser Leu Gly Gly Lys Glu Glu Phe Val Ala Thr Thr Glu
   705 710 715 720
   Ser Thr Thr Glu Thr Lys Glu Pro Lys Lys Ala Lys Arg Ser Ser Lys
   725 730 735
   Glu Glu Ala Glu Met Ala Tyr Lys Asp Phe Leu Leu Gln Ser Ser Thr
   740 745 750
   Val Ala Ala Glu Ala Gln Asp Gly Pro Gln Glu Ala
   755 760
<210> 8
   <211> 760
   <212> PRT
   <213> Homo sapiens
<400> 8
   Met Met Asp Gln Ala Arg Ser Ala Phe Ser Asn Leu Phe Gly Gly Glu
   1               5                   10                  15
   Pro Leu Ser Tyr Thr Arg Phe Ser Leu Ala Arg Gln Val Asp Gly Asp
               20                  25                  30
   Asn Ser His Val Glu Met Lys Leu Ala Val Asp Glu Glu Glu Asn Ala
           35                  40                  45
   Asp Asn Asn Thr Lys Ala Asn Val Thr Lys Pro Lys Arg Cys Ser Gly
       50                  55                  60
   Ser Ile Cys Tyr Gly Thr Ile Ala Val Ile Val Phe Phe Leu Ile Gly
   65                  70                  75                  80
   Phe Met Ile Gly Tyr Leu Gly Tyr Cys Lys Gly Val Glu Pro Lys Thr
                   85                  90                  95
   Glu Cys Glu Arg Leu Ala Gly Thr Glu Ser Pro Val Arg Glu Glu Pro
               100                 105                 110
   Gly Glu Asp Phe Pro Ala Ala Arg Arg Leu Tyr Trp Asp Asp Leu Lys
           115                 120                 125
   Arg Lys Leu Ser Glu Lys Leu Asp Ser Thr Asp Phe Thr Ser Thr Ile
       130                 135                 140
   Lys Leu Leu Asn Glu Asn Ser Tyr Val Pro Arg Glu Ala Gly Ser Gln
   145                 150                 155                 160
   Lys Asp Glu Asn Leu Ala Leu Tyr Val Glu Asn Gln Phe Arg Glu Phe
                   165                 170                 175
   Lys Leu Ser Lys Val Trp Arg Asp Gln His Phe Val Lys Ile Gln Val
               180                 185                 190
   Lys Asp Ser Ala Gln Asn Ser Val Ile Ile Val Asp Lys Asn Gly Arg
           195                 200                 205
   Leu Val Tyr Leu Val Glu Asn Pro Gly Gly Tyr Val Ala Tyr Ser Lys
       210                 215                 220
   Ala Ala Thr Val Thr Gly Lys Leu Val His Ala Asn Phe Gly Thr Lys
   225 230 235 240
   Lys Asp Phe Glu Asp Leu Tyr Thr Pro Val Asn Gly Ser Ile Val Ile
   245 250 255
   Val Arg Ala Gly Lys Ile Thr Phe Ala Glu Lys Val Ala Asn Ala Glu
   260 265 270
   Ser Leu Asn Ala Ile Gly Val Leu Ile Tyr Met Asp Gln Thr Lys Phe
   275 280 285
   Pro Ile Val Asn Ala Glu Leu Ser Phe Phe Gly His Ala His Leu Gly
   290 295 300
   Thr Gly Asp Pro Tyr Thr Pro Gly Phe Pro Ser Phe Asn His Thr Gln
   305 310 315 320
   Phe Pro Pro Ser Arg Ser Ser Gly Leu Pro Asn Ile Pro Val Gln Thr
   325 330 335
   Ile Ser Arg Ala Ala Ala Glu Lys Leu Phe Gly Asn Met Glu Gly Asp
   340 345 350
   Cys Pro Ser Asp Trp Lys Thr Asp Ser Thr Cys Arg Met Val Thr Ser
   355 360 365
   Glu Ser Lys Asn Val Lys Leu Thr Val Ser Asn Val Leu Lys Glu Ile
   370 375 380
   Lys Ile Leu Asn Ile Phe Gly Val Ile Lys Gly Phe Val Glu Pro Asp
   385 390 395 400
   His Tyr Val Val Val Gly Ala Gln Arg Asp Ala Trp Gly Pro Gly Ala
   405 410 415
   Ala Lys Ser Gly Val Gly Thr Ala Leu Leu Leu Lys Leu Ala Gln Met
   420 425 430
   Phe Ser Asp Met Val Leu Lys Asp Gly Phe Gln Pro Ser Arg Ser Ile
   435 440 445
   Ile Phe Ala Ser Trp Ser Ala Gly Asp Phe Gly Ser Val Gly Ala Thr
       450                 455                 460
   Glu Trp Leu Glu Gly Tyr Leu Ser Ser Leu His Leu Lys Ala Phe Thr
   465                 470                 475                 480
   Tyr Ile Asn Leu Asp Lys Ala Val Leu Gly Thr Ser Asn Phe Lys Val
                   485                 490                 495
   Ser Ala Ser Pro Leu Leu Tyr Thr Leu Ile Glu Lys Thr Met Gln Asn
               500                 505                 510
   Val Lys His Pro Val Thr Gly Gln Phe Leu Tyr Gln Asp Ser Asn Trp
           515                 520                 525
   Ala Ser Lys Val Glu Lys Leu Thr Leu Asp Asn Ala Ala Phe Pro Phe
       530                 535                 540
   Leu Ala Tyr Ser Gly Ile Pro Ala Val Ser Phe Cys Phe Cys Glu Asp
   545                 550                 555                 560
   Thr Asp Tyr Pro Tyr Leu Gly Thr Thr Met Asp Thr Tyr Lys Glu Leu
                   565                 570                 575
   Ile Glu Arg Ile Pro Glu Leu Asn Lys Val Ala Arg Ala Ala Ala Glu
               580                 585                 590
   Val Ala Gly Gln Phe Val Ile Lys Leu Thr His Asp Val Glu Leu Asn
           595                 600                 605
   Leu Asp Tyr Glu Arg Tyr Asn Ser Gln Leu Leu Ser Phe Val Arg Asp
       610                 615                 620
   Leu Asn Gln Tyr Arg Ala Asp Ile Lys Glu Met Gly Leu Ser Leu Gln
   625                 630                 635                 640
   Trp Leu Tyr Ser Ala Arg Gly Asp Phe Phe Arg Ala Thr Ser Arg Leu
                   645                 650                 655
   Thr Thr Asp Phe Gly Asn Ala Glu Lys Thr Asp Arg Phe Val Met Lys
               660                 665                 670
   Lys Leu Asn Asp Arg Val Met Arg Val Glu Tyr His Phe Leu Ser Pro
   675 680 685
   Tyr Val Ser Pro Lys Glu Ser Pro Phe Arg His Val Phe Trp Gly Ser
   690 695 700
   Gly Ser His Thr Leu Pro Ala Leu Leu Glu Asn Leu Lys Leu Arg Lys
   705 710 715 720
   Gln Asn Asn Gly Ala Phe Asn Glu Thr Leu Phe Arg Asn Gln Leu Ala
   725 730 735
   Leu Ala Thr Trp Thr Ile Gln Gly Ala Ala Asn Ala Leu Ser Gly Asp
   740 745 750
   Val Trp Asp Ile Asp Asn Glu Phe
   755 760
<210> 9
   <211> 9
   <212> PRT
   <213> Artificial sequence
<220>
   <223> IgM binding peptide
<400> 9
   Tyr Asp Trp Ile Pro Ser Ser Ala Trp
   1 5
<210> 10
   <211> 273
   <212> PRT
   <213> Staphylococcus aureus
<400> 10
   Met Glu Gln Arg Ile Thr Leu Lys Glu Ala Trp Asp Gln Arg Asn Gly
   1 5 10 15
   Phe Ile Gln Ser Leu Lys Asp Asp Pro Ser Gln Ser Ala Asn Val Leu
   20 25 30
   Gly Glu Ala Gln Lys Leu Asn Asp Ser Gln Ala Pro Lys Ala Asp Ala
   35 40 45
   Gln Gln Asn Asn Phe Asn Lys Asp Gln Gln Ser Ala Phe Tyr Glu Ile
   50 55 60
   Leu Asn Met Pro Asn Leu Asn Glu Ala Gln Arg Asn Gly Phe Ile Gln
   65 70 75 80
   Ser Leu Lys Asp Asp Pro Ser Gln Ser Thr Asn Val Leu Gly Glu Ala
   85 90 95
   Lys Lys Leu Asn Glu Ser Gln Ala Pro Lys Ala Asp Asn Asn Phe Asn
   100 105 110
   Lys Glu Gln Gln Asn Ala Phe Tyr Glu Ile Leu Asn Met Pro Asn Leu
   115 120 125
   Asn Glu Glu Gln Arg Asn Gly Phe Ile Gln Ser Leu Lys Asp Asp Pro
   130 135 140
   Ser Gln Ser Ala Asn Leu Leu Ser Glu Ala Lys Lys Leu Asn Glu Ser
   145 150 155 160
   Gln Ala Pro Lys Ala Asp Asn Lys Phe Asn Lys Glu Gln Gln Asn Ala
   165 170 175
   Phe Tyr Glu Ile Leu His Leu Pro Asn Leu Asn Glu Glu Gln Arg Asn
   180 185 190
   Gly Phe Ile Gln Ser Leu Lys Asp Asp Pro Ser Gln Ser Ala Asn Leu
   195 200 205
   Leu Ala Glu Ala Lys Lys Leu Asn Asp Ala Gln Ala Pro Lys Ala Asp
   210 215 220
   Asn Lys Phe Asn Lys Glu Gln Gln Asn Ala Phe Tyr Glu Ile Leu His
   225 230 235 240
   Leu Pro Asn Leu Thr Glu Glu Gln Arg Asn Gly Phe Ile Gln Ser Leu
   245 250 255
   Lys Asp Asp Pro Gly Asn Ser Arg Gly Ser Val Asp Leu Gln Ile Thr
   260 265 270
   Asn
<210> 11
   <211> 58
   <212> PRT
   <213> Artificial Sequence
<220>
   <223> Z-domain
<400> 11
<210> 12
   <211> 58
   <212> PRT
   <213> Artificial Sequence
<220>
   <223> Modified Z-domain
<400> 12
   Val Asp Asn Lys Phe Asn Lys Glu Thr Ile Gln Ala Ser Gln Glu Ile
   1 5 10 15
   Arg Leu Leu Pro Asn Leu Asn Gly Arg Gln Lys Leu Ala Phe Ile His
   20 25 30
   Ser Leu Leu Asp Asp Pro Ser Gln Ser Ala Asn Leu Leu Ala Glu Ala
   35 40 45
   Lys Lys Leu Asn Asp Ala Gln Ala Pro Lys
   50 55
<210> 13
   <211> 58
   <212> PRT
   <213> Staphylococcus aureus
<400> 13
<210> 14
   <211> 25
   <212> PRT
   <213> Homo sapiens
<400> 14
   Thr Val Pro Cys Pro Val Pro Ser Thr Pro Pro Thr Pro Ser Pro Ser
   1 5 10 15
   Thr Pro Pro Thr Pro Ser Pro Ser Cys
   20 25
<210> 15
   <211> 627
   <212> PRT
   <213> Homo sapiens
<400> 15
<210> 16
   <211> 2213
   <212> DNA
   <213> Homo sapiens
<400> 16
   gctctagaac tagtggatcc cccgggctgc aggaattctc taaagaagcc cctgggagca 60
   cagctcatca ccatggactg gacctggagg ttcctctttg tggtggcagc agctacaggt 120
   gtccagtccc aggtgcagct ggtgcagtct ggggctgagg tgaagaagcc tgggtcctcg 180
   gtgaaggtct cctgcaaggc ttctggaggc accttcagca gctatgctat cagctgggtg 240
   cgacaggccc ctggacaagg gcttgagtgg atgggaggga tcatccctat ctttggtaca 300
   gcaaactacg cacagaagtt ccagggcaga gtcacgatta ccgcggacga atccacgagc     360
   acagcctaca tggagctgag cagcctgaga tctgaggaca cggccgtgta ttactgtgcg     420
   aaaaccggga tcctggggcc gtatagcagt ggctggtacc cgaactcgga ctactactac     480
   tacggtatgg acgtctgggg ccaagggacc acggtcaccg tctcctcagg gagtgcatcc     540
   gccccaaccc ttttccccct cgtctcctgt gagaattccc cgtcggatac gagcagcgtg    600
   gccgttggct gcctcgcaca ggacttcctt cccgactcca tcactttctc ctggaaatac     660
   aagaacaact ctgacatcag cagcacccgg ggcttcccat cagtcctgag agggggcaag     720
   tacgcagcca cctcacaggt gctgctgcct tccaaggacg tcatgcaggg cacagacgaa     780
   cacgtggtgt gcaaagtcca gcaccccaac ggcaacaaag aaaagaacgt gcctcttcca     840
   gtgattgctg agctgcctcc caaagtgagc gtcttcgtcc caccccgcga cggcttcttc     900
   ggcaaccccc gcagcaagtc caagctcatc tgccaggcca cgggtttcag tccccggcag     960
   attcaggtgt cctggctgcg cgaggggaag caggtggggt ctggcgtcac cacggaccag    1020
   gtgcaggctg aggccaaaga gtctgggccc acgacctaca aggtgaccag cacactgacc    1080
   atcaaagaga gcgactggct cagccagagc atgttcacct gccgcgtgga tcacaggggc    1140
   ctgaccttcc agcagaatgc gtcctccatg tgtgtccccg atcaagacac agccatccgg    1200
   gtcttcgcca tccccccatc ctttgccagc atcttcctca ccaagtccac caagttgacc   1260
   tgcctggtca cagacctgac cacctatgac agcgtgacca tctcctggac ccgccagaat    1320
   ggcgaagctg tgaaaaccca caccaacatc tccgagagcc accccaatgc cactttcagc    1380
   gccgtgggtg aggccagcat ctgcgaggat gactggaatt ccggggagag gttcacgtgc    1440
   accgtgaccc acacagacct gccctcgcca ctgaagcaga ccatctcccg gcccaagggg    1500
   gtggccctgc acaggcccga tgtctacttg ctgccaccag cccgggagca gctgaacctg    1560
   cgggagtcgg ccaccatcac gtgcctggtg acgggcttct ctcccgcgga cgtcttcgtg    1620
   cagtggatgc agagggggca gcccttgtcc ccggagaagt atgtgaccag cgccccaatg    1680
   cctgagcccc aggccccagg ccggtacttc gcccacagca tcctgaccgt gtccgaagag    1740
   gaatggaaca cgggggagac ctacacctgc gtggtggccc atgaggccct gcccaacagg    1800
   gtcaccgaga ggaccgtgga caagtccacc gagggggagg tgagcgccga cgaggagggc    1860
   tttgagaacc tgtgggccac cgcctccacc ttcatcgtcc tcttcctcct gagcctcttc    1920
   tacagtacca ccgtcacctt gttcaaggtg aaatgatccc aacagaagaa catcggagac    1980
   cagagagagg aactcaaagg ggcgctgcct ccgggtctgg ggtcctggcc tgcgtggcct 2040
   gttggcacgt gtttctcttc ccgcccggcc tccagttgtg tgctctcaca caggcttcct 2100
   tctcgaccgg caggggctgg ctggcttgca ggccacgagg tgggctctac cccacactgc 2160
   tttgctgtgt atacgcttgt tgccctgaaa taaatatgca cattttatcc atg 2213
<210> 17
   <211> 26
   <212> PRT
   <213> Homo sapiens
<400> 17
   Pro Leu Pro Val Ile Ala Glu Leu Pro Pro Lys Val Ser Val Phe Val
   1 5 10 15
   Pro Pro Arg Asp Gly Phe Phe Gly Asn Pro
   20 25